# EUROPEAN PATENT SPECIFICATION

(11) **EP 4 116 428 B1**
(45) Date of publication and mention of the grant of the patent: **12.11.2025**
(21) Application number: 21760911.4
(22) Date of filing: 26.02.2021
(51) Int. Cl.: C12P 7/62, C08G 63/06, C12P 7/625

(54) **BLOCK COPOLYMER AND METHOD FOR PRODUCING SAME**
BLOCK-COPOLYMER UND VERFAHREN ZUR HERSTELLUNG DAVON
COPOLYMÈRE SÉQUENCÉ ET SON PROCÉDÉ DE PRODUCTION

(30) Priority: 28.02.2020 JP 2020034109
(43) Date of publication of application: 11.01.2023
(73) Proprietor: National University Corporation Hokkaido University, Sapporo-shi, Hokkaido 060-0808 (JP)
(72) Inventor: MATSUMOTO, Kenichiro, Sapporo-shi, Hokkaido 060-0808 (JP); FUKUI, Toshiaki, Tokyo 152-8550 (JP); ISHIHARA, Shizuru, Tokyo 152-8550 (JP)
(74) Representative: J A Kemp LLP
(86) International application number: PCT/JP2021/007499
(87) International publication number: WO 2021/172559

(56) References cited:
- US-A1- 2008 275 208
- ARAI SHUZO ET AL: "Biosynthesis of Random-Homo Block Copolymer Poly[Glycolate-ran-3-Hydroxybutyrate (3HB)]-b-Poly(3HB) Using Sequence-Regulating Chimeric Polyhydroxyalkanoate Synthase in Escherichia coli", FRONTIERS IN BIOENGINEERING AND BIOTECHNOLOGY, vol. 8, 8 December 2020 (2020-12-08), pages 1 - 10, XP055850473, DOI: 10.3389/fbioe.2020.612991
- ARAI SHUZO ET AL: "Dynamic Changes of Intracellular Monomer Levels Regulate Block Sequence of Polyhydroxyalkanoates in Engineered Escherichia coli", vol. 19, no. 2, 12 February 2018 (2018-02-12), US, pages 662 - 671, XP055850465, ISSN: 1525-7797, Retrieved from the Internet <URL:https://pubs.acs.org/doi/pdf/10.1021/acs.biomac.7b01768> [retrieved on 20230821], DOI: 10.1021/acs.biomac.7b01768
- JIAN SUN ET AL: "Production of P(3-hydroxybutyrate- co -3-hydroxyhexanoate- co -3-hydroxyoctanoate) Terpolymers Using a Chimeric PHA Synthase in Recombinant Ralstonia eutropha and Pseudomonas putida", BIOSCIENCE, BIOTECHNOLOGY, AND BIOCHEMISTRY, vol. 74, no. 8, 23 August 2010 (2010-08-23), JP, pages 1716 - 1718, XP055358664, ISSN: 0916-8451, DOI: 10.1271/bbb.100242
- MATSUMOTO KEN’ICHIRO, HORI CHIAKI, FUJII RYUNOSUKE, TAKAYA MASAHIRO, OOBA TAKASHI, OOI TOSHIHIKO, ISONO TAKUYA, SATOH TOSHIFUMI, T: "Dynamic Changes of Intracellular Monomer Levels Regulate Block Sequence of Polyhydroxyalkanoates in Engineered Escherichia coli", BIOMACROMOLECULES, vol. 19, no. 2, 12 February 2018 (2018-02-12), US, pages 662 - 671, XP055850465, ISSN: 1525-7797, DOI: 10.1021/acs.biomac.7b01768
- MATSUMOTO, KENICHIRO: "Development on Biosynthesis of sequence-regulated polyesters", BAIOSAIENSU TO INDASUTORI - BIOSCIENCE TO INDUSTRY, vol. 77, no. 3, 10 May 2019 (2019-05-10), JP, pages 252 - 253, XP009530593, ISSN: 0914-8981
- SUN, J. ET AL.: "Production of P(3- hydroxybutyrate-co-3-hydroxyhexanoate-co-3- hydroxyoctanoate) terpolymers using a chimeric PHA synthase in recombinant Ralstonia eutropha and Pseudomonas putida", BIOSCIENCE, BIOTECHNOLOGY, AND BIOCHEMISTRY, vol. 74, no. 8, 7 August 2010 (2010-08-07), pages 1716 - 1718, XP055358664, DOI: 10.1271/bbb.100242
- ARAI, S. ET AL.: "Biosynthesis of Random-Homo Block Copolymer Poly [Glycolate-ran-3- Hydroxybutyrate (3HB)]-b-Poly(3HB) Using Sequence- Regulating Chimeric Polyhydroxyalkanoate Synthase in Escherichia coli", FRONTIERS IN BIOENGINEERING AND BIOTECHNOLOGY, vol. 8, no. 612991, 8 December 2020 (2020-12-08), pages 1 - 10, XP055850473
- MENG DE-CHUAN, CHEN GUO-QIANG: "Synthetic Biology of Polyhydroxyalkanoates (PHA", ADVANCES IN BIOCHEMICAL ENGINEERING/BIOTECHNOLOGY, vol. 162, 2018, DE, pages 147 - 174, XP009530595, ISSN: 0724-6145, DOI: 10.1007/10_2017_3

## Description

### [Technical Field]

The present invention relates to a block copolymer and a method for producing the same, and more specifically relates to a block copolymer having a homopolymer segment and a copolymer segment and a method for producing the same.

### [Background Art]

Copolymers are polymer compounds synthesized by polymerizing two or more types of monomers, and are excellent in physical properties such as elasticity, transparency, processability, and light weight as compared with the other materials such as metals. For this reason, copolymers are frequently used in various industrial fields such as the medical field. Among those, block copolymers composed of chains in which two or more types of polymers (segments) having different characteristics are covalently bound are expected to be materials having the features of the respective constituent segments.

In addition, such polymer compounds are normally synthesized using petroleum as raw materials. However, in order to deal with depletion of fossil resources and global warming, which have been controversial these days, polymer compounds (so-called biopolymers) that are produced using renewable resources (raw materials derived from biomass) as carbon sources have been attracting attention.

For example, polyhydroxyalkanoates (PHAs) produced by a microorganism have been drawing attention as bio-based biodegradable materials. A PHA currently commercially produced is a copolymer of 3-hydroxybutyrate (3HB) and 3-hydroxyhexanoic acid (3HHx). Moreover, since the polymerization of non-natural lactate units was reported in 2008, PHAs containing various non-natural monomers have been reported (NPL 1).

In addition, in a microorganism, two or more types of monomers are sequenced normally at random, and PHAs are biosynthesized as random copolymers. However, the present inventors reported that a block copolymer (P(2HB-b-3HB)) in which a homopolymer segment composed of 2-hydroxybutyric acid (2HB) and a homopolymer segment composed of 3HB were bound was successfully biosynthesized by culturing a microorganism expressing CoA transferase and polymerizing enzyme in a medium containing 2-hydroxybutyric acid (2HB) and 3HB (NPL 2).

As described above, regarding biopolymers as well, while the biosynthesis of block copolymers is becoming possible, the development of further block copolymers has been demanded for improving physical properties and the like.

NPL3 describes the production of P(3-hydroxybutyrate-co-3-hydroxyhexanoate-co-3-hydroxyactanoate) terpolymers using a chimeric PHA synthase in recombinant Ralstonia eutropha and Pseudomonas putida. This is said to produce 3-hydroxybutyrate (3HB)-based PHA copolymers comprised of 3-hydroxyhexanoate and 3-hydroxyoctanoate units from dodecanoate (87-97 mol% 3HB).

PL 1 describes PHA copolymer compositions produced using biological systems which include monomers such as 3-hydroxybutyrate, 3-hydroxypropionate, 2-hydroxybutyrate, 3-hydroxyvalerate, 4-hydroxybutyrate, 4-hydroxyvalerate and 5-hydroxyvalerate. The PHA compositions are said to be readily extended to incorporate additional monomers including 3-hydroxyheaxanoate, 4-hydroxyhexanoate, 6-hydroxyhexanoate or other longer chain 3-hydroxyacids containing seven or more carbons. This is said to be accomplished by taking natural PHA producers and mutating through chemical or transposon mutagenesis to delete or inactive genes encoding undesirable activities.

### [Citation List]

### [Non Patent Literature]

[NPL 1] Matsumoto et al., 2013, Appl. Microbiol. Biotechnol. 97, 8011
[NPL 2] Matsumoto K et al., Biomacromolecules, 19(2), 662, 2018
[NPL3] Sun et al., Bioscience, Biotechnology, and Biochemistry, 74(8), 1716-1718, 2010

### [Patent Literature]

[PL1] US 2008 0275208 A1

### [Summary of Invention]

### [Technical Problem]

The present invention has been made in view of the above-described problems of the conventional techniques, and an object thereof is to provide a block copolymer having a homopolymer segment and a copolymer segment, a segment being a structural unit which constitutes each block in a block copolymer and which is a unit composed of one type of monomer or a unit composed of a plurality of types of monomers.

### [Solution to Problem]

The present inventors cultured the above-described microorganism in a medium in which 3HHx was also added in addition to the previous 2HB and 3HB in order to further improve the block copolymer (P(2HB-b-3HB)) reported in NPL 2. As a result, a block copolymer (P(2HB-b-(3HB-co-3HHx))) in which a homopolymer segment composed of 2HB and a random polymer segment composed of 3HB and 3HHx were bound was successfully obtained. That is, a block copolymer containing also a random copolymer segment, which was different from the previous block copolymer containing only homopolymer segments, was successfully biosynthesized.

Moreover, it was confirmed that a block copolymer (P(2HB)-b-P(3HB-ran-3HP), P(2HB)-b-P(3HB-ran-4H2MB), P(2HB)-b-P(3HB-ran-24DHB), P(2HB)-b-P(3HB-ran-5HV), or P(2HB)-b-P(3HB-ran-6HHx)) was successfully synthesized as in the case of using 3HHx, by adding 3-hydroxypropionic acid (3HP), 4-hydroxy-2-methylbutyric acid (4H2MB), 2,4-dihydroxybutyric acid (24DHB), 5-hydroxyvaleric acid (5HV), or 6-hydroxyhexanoic acid (6HHx) instead of 3HHx to a medium containing 2HB and 3HB, and culturing the microorganism in the medium.

In addition, as a result of culturing the microorganism in a medium to which 2-hydroxyacetic acid (glycolic acid, GL) and 3HB were added, a block copolymer (P(3HB-b-(GL-co-3HB))) in which a homopolymer segment composed of 3HB and a copolymer segment composed of GL and 3HB were bound was successfully obtained.

Furthermore, as a result of culturing the microorganism in a medium to which GL, 3HB, and 3HHx were added, a block copolymer (P(3HB-b-(GL-co-3HB-co-3HHx))) in which a homopolymer segment composed of 3HB and a random copolymer segment composed of GL, 3HB, and 3HHx were bound was successfully obtained.

In addition, it was revealed that in the case where a Ralstonia eutropha glucose-utilizing modified strain in which ldhA (lactate dehydrogenase gene), hadA (CoA transferase gene), and PhaC_{AR} (polymerizing enzyme gene) were introduced was cultured in a medium containing glucose and 2HB, this microorganism biosynthesized 3HB and3HV (3-hydroxyvaleric acid), and a block copolymer (P(2HB)-b-P(3HB-co-3HV)) was also successfully biosynthesized from these hydroxycarboxylic acids. That is, the above block copolymer was successfully obtained by culturing the above microorganism without adding hydroxycarboxylic acids having hydroxy groups at positions other than the 2-position such as 3HB and 3HV to the medium.

The present invention is based on the above-described synthesis examples. More specifically, the invention is as set out in the claims.

### [Advantageous Effects of Invention]

The present invention makes it possible to provide a block copolymer having a homopolymer segment and a copolymer segment and a method for producing the same, a segment being a structural unit which constitutes each block in a block copolymer and which is a unit composed of one type of monomer or a unit composed of a plurality of types of monomers.

The present invention can provide, for example, a block copolymer (P(2HB-b-(3HB-co-3HHx))) in which a homopolymer composed of 2HB and a random polymer composed of 3HB and 3HHx are bound. As shown in Examples described later, this block copolymer has a higher extensibility than the copolymer P(2HB-b-3HB) disclosed in NPL 2. In particular, P(2HB-b-(3HB-co-3HHx)) has a significantly high extensibility of more than 1200%.

In addition, the present invention can provide a block copolymer (P(3HB-b-(GL-co-3HB))) in which a homopolymer composed of 3HB and a copolymer composed of GL and 3HB are bound. Moreover, the present invention can provide a block copolymer (P(3HB-b-(GL-co-3HB-co-3HHx))) in which a homopolymer composed of 3HB and a copolymer composed of GL, 3HB, and 3HHx are bound. As shown in Examples described later, these block copolymers have improved non-enzymatic hydrolyzability, and thus exhibit high degradability in vivo because GL is introduced. In addition, the copolymer segment of P(3HB-b-(GL-co-3HB-co-3HHx)) takes a gradient structure. Moreover, this copolymer has a significantly improved flexibility because 3HHx is introduced.

### [Brief Description of Drawings]

[Fig. 1A] Fig. 1A is a chart showing a result of ¹H NMR spectroscopy on a copolymer (P(2HB-b-(3HB-co-3HHx))) obtained by charging 2HB, 3HB, and 3HHx and using a polymerizing enzyme PhaC_{AR}.
[Fig. 1B] Fig. 1B is a chart showing a result of ¹³C NMR spectroscopy on a copolymer (P(2HB-b-(3HB-co-3HHx))) obtained by charging 2HB, 3HB and 3HHx and using the polymerizing enzyme PhaC_{AR}.
[Fig. 1C] Fig. 1C is a plot showing a stress-strain relation regarding P(2HB-b-(3HB-co-3HHx)) and the like.
[Fig. 2A] Fig. 2A is a chart showing a result of ¹H NMR spectroscopy on a copolymer obtained by charging 3HB and GL and using a polymerizing enzyme PhaC1_{Ps}STQK.
[Fig. 2B] Fig. 2B is a chart showing a result of ¹H NMR spectroscopy on a copolymer (P(3HB-b-(GL-co-3HB))) obtained by charging 3HB and GL and using the polymerizing enzyme PhaC_{AR}.
[Fig. 2C] Fig. 2C is a molecular weight distribution curve showing a result of analyzing P(3HB-b-(GL-co-3HB)) by using gel filtration chromatography.
[Fig. 3A] Fig. 3A is a chart showing a result of ¹H NMR spectroscopy on P(3HB-b-(GL-co-3HB-co-3HHx)). Note that numbers in Fig. 3A correspond to description of Table 2.
[Fig. 3B] Fig. 3B is a graph showing a result of differential scanning calorimetry on P(3HB-b-(GL-co-3HB-co-3HHx)).
[Fig. 3C] Fig. 3C is a plot showing a stress-strain relation regarding P(3HB-b-(GL-co-3HB-co-3HHx)).
[Fig. 4A] Fig. 4A is a chart showing a result of conducting ¹H NMR spectroscopy on a block copolymer composed of a P(2HB) homopolymer segment and a random copolymer segment containing 3HB.
[Fig. 4B] Fig. 4B is a graph showing a result of differential scanning calorimetry on P(2HB)-b-P(3HB-ran-5HV) .
[Fig. 5A] Fig. 5A is a schematic diagram showing a metabolic pathway for biosynthesizing P(2HB)-b-P(3HB-co-3HV).
[Fig. 5B] Fig. 5B is a schematic diagram showing that in the pha operon on the chromosome of the R. eutrpha H16 NSDG-GG-ΔB1 strain, phaCAR (a chimeric PHA synthase gene derived from A.caviae and R.eutropha) was substituted for PhaCNSDG (Aeromonas caviae-derived modified PHA synthase gene) through homologous recombination.
[Fig. 5C] Fig. 5C is a schematic diagram showing that Clostridium difficile-derived ldhA (lactate dehydrogenase gene) and hadA (CoA transferase gene) were inserted downstream of phaP1 (PHA granule-binding protein gene).
[Fig. 5D] Fig. 5D is a chart showing a result of conducting ¹H NMR spectroscopy on P(2HB)-b-P(3HB-co-3HV).

### [Description of Embodiments]

### <Block copolymer>

As shown in Examples described below, the present invention provides a block copolymer comprising a homopolymer segment and a copolymer segment, and more specifically provides
a copolymer comprising: a hydroxycarboxylic acid (A) having a hydroxy group only at a 2-position; and a hydroxycarboxylic acid (B) having a hydroxy group at a position other than a 2-position, and having
a homopolymer segment composed of one hydroxycarboxylic acid selected from the group consisting of the hydroxycarboxylic acid (A) and the hydroxycarboxylic acid (B), and
a copolymer segment containing at least two hydroxycarboxylic acids selected from the group consisting of the hydroxycarboxylic acid (A) and the hydroxycarboxylic acid (B).

The copolymer of the present invention is obtained by polymerizing hydroxycarboxylic acids through ester bonds, and has at least two types of segments.

In the present invention, the "segment" is a structural unit which constitutes each block in a block copolymer, and is a unit composed of one type of monomer (a hydroxycarboxylic acid described later) (a homopolymer segment) or a unit composed of a plurality of types of monomers (a copolymer segment). The "copolymer segment" includes, for example, a random polymer segment in which a plurality of types of monomers are polymerized at random, a gradient polymer segment in which the composition of monomers included continuously changes, an alternate polymer segment in which a plurality of types of monomers are polymerized alternately. In addition, the number of monomers contained in each segment in the copolymer of the present invention is not particularly limited, but when the copolymer is used as a material, the number of monomers is preferably 100 or more, and normally 500 or more, and more preferably 4000 or more.

The "hydroxycarboxylic acid" according to the present invention is divided into two types, a monohydroxycarboxylic acid having a hydroxy group only at a 2-position and a hydroxycarboxylic acid having a hydroxy group at a position other than a 2-position. Note that the latter may be a hydroxycarboxylic acid having a plurality of hydroxy groups, and in this case, the hydroxycarboxylic acid may have a hydroxy group at the 2-position in addition to the position other than the 2-position.

The hydroxycarboxylic acid having a hydroxy group only at the 2-position (hereinafter also referred to as the "hydroxycarboxylic acid (A)") includes, for example, 2-hydroxyacetic acid (glycolic acid, GL), 2-hydroxybutyric acid (2HB), 2-hydroxypropionic acid (lactate, 2HP), 2-hydroxypentanoic acid (2-hydroxyvaleric acid), 2-hydroxyhexanoic acid, and 2-hydroxyalkanoic acids derived from amino acids (derived from side chain structures of amino acids) (see Sudo M. et al., J Biosci Bioeng. 2019 Oct 18. pii: S1389-1723(19) 30785-6). In addition, the hydroxycarboxylic acid (A) according to the present invention means not only one type of monohydroxycarboxylic acid having a hydroxy group only at the 2-position, but also monohydroxycarboxylic acids having hydroxy groups only at a plurality of types of 2-positions. Among these, 2-hydroxyacetic acid is preferable from the viewpoint that when introduced into the copolymer, 2-hydroxyacetic acid improves the non-enzymatic hydrolyzability, and thus makes it easy to exhibit a high degradability in vivo. Moreover, 2-hydroxybutyric acid is preferable from the viewpoint that 2-hydroxybutyric acid provides the material with transparency, makes it possible to obtain an isotactic polymer having a tacticity similar to poly-D-lactic acid (PDLA), and facilitates biosynthesis because the glass transition temperature of the polymer is lower than the other 2-hydroxycarboxylic acids.

The hydroxycarboxylic acid having a hydroxy group at a position other than the 2-position (hereinafter also referred to as the "hydroxycarboxylic acid (B)") includes, for example, 3-hydroxypropionic acid (3HP), 3-hydroxybutyrate (3HB), 3-hydroxyhexanoic acid (3HHx), 3-hydroxypentanoic acid (3-hydroxyvaleric acid, 3HV), 4-hydroxy-2-methylbutyric acid (4H2MB), 4-hydroxybutyric acid, 2,4-dihydroxybutyric acid (24DHB), 5-hydroxypentanoic acid (5-hydroxyvaleric acid, 5HV), and 6-hydroxyhexanoic acid (6HHx). Note that the hydroxycarboxylic acid (B) according to the present invention means not only one type of hydroxycarboxylic acid having a hydroxy group at a position other than the 2-position, but also hydroxycarboxylic acids having hydroxy groups at positions other than a plurality of types of 2-positions. Among these, 3-hydroxybutyrate is preferable from the viewpoint that 3-hydroxybutyrate can most efficiently be synthesized from various carbon sources and provides the material with strength, and 3-hydroxyhexanoic acid is preferable from the viewpoint that the flexibility is improved by introducing 3-hydroxyhexanoic acid into the copolymer.

In the present invention, the combination of these hydroxycarboxylic acid (A) and hydroxycarboxylic acid (B) is not particularly limited as long as it is possible to form the above-mentioned 2 types of segments, and the combination includes, for example, 2-hydroxybutyric acid, 3-hydroxybutyrate, and 3-hydroxyhexanoic acid; 2-hydroxyacetic acid and 3-hydroxybutyrate; 2-hydroxyacetic acid, 3-hydroxybutyrate and 3-hydroxyhexanoic acid; 2-hydroxybutyric acid, 3-hydroxybutyrate, and 3-hydroxypropionic acid; 2-hydroxybutyric acid, 3-hydroxybutyrate, and 4-hydroxy-2-methylbutyric acid; 2-hydroxybutyric acid, 3-hydroxybutyrate, and 2,4-dihydroxybutyric acid; 2-hydroxybutyric acid, 3-hydroxybutyrate, and 5-hydroxyvaleric acid; 2-hydroxybutyric acid, 3-hydroxybutyrate, and 6-hydroxyhexanoic acid; 2-hydroxybutyric acid, 3-hydroxypropionic acid, and 4-hydroxy-2-methylbutyric acid; 2-hydroxybutyric acid, 3-hydroxypropionic acid, and 2,4-dihydroxybutyric acid; 2-hydroxybutyric acid, 3-hydroxypropionic acid, and 5-hydroxyvaleric acid; 2-hydroxybutyric acid, 3-hydroxypropionic acid, and 6-hydroxyhexanoic acid; and 2-hydroxybutyric acid, 3-hydroxybutyrate, and 3-hydroxyvaleric acid, but the combination of 2-hydroxybutyric acid and 3-hydroxybutyrate is excluded.

In addition, the molecular weight of the copolymer of the present invention composed of such a combination is not particularly limited, but is preferably 10,000 or more, more preferably 100,000 or more, and further preferably 1,000,000 or more, in terms of the number average molecular weight, from the viewpoint of obtaining a sufficient mechanical strength and further keeping a sufficient molecular weight even after the molecular weight is reduced by melt molding. Moreover, the upper limit is also not particularly limited, but is normally 10,000,000 or less from the viewpoint that otherwise the production becomes difficult.

Hereinafter, the copolymer of the present invention will be further described based on specific examples of combinations.

In the copolymer of the present invention, a copolymer comprising 2-hydroxybutyric acid, 3-hydroxybutyrate, and 3-hydroxyhexanoic acid has a homopolymer segment composed of 2-hydroxybutyric acid, and a copolymer segment containing 3-hydroxybutyrate and 3-hydroxyhexanoic acid. Note that the copolymer segment in this copolymer takes a random copolymer structure.

The molecular weight (number average molecular weight) of this copolymer is not particularly limited, but is preferably 100,000 or more, and more preferably 1,000,000 or more, from the viewpoint of obtaining a sufficient mechanical strength and further keeping a sufficient molecular weight even after the molecular weight is reduced by melt molding.

In addition, the content of the random copolymer segment of 3-hydroxybutyrate and 3-hydroxyhexanoic acid is preferably 50 mol% or more, and more preferably 70 mol% or more of the entire polymer, from the viewpoint of exhibiting a sufficient flexibility and exhibiting physical properties originating from the block structure. The composition of this random segment is such that the content of 3-hydroxyhexanoic acid is preferably 5 mol% or more, and more preferably 20 mol% or more, from the viewpoint of exhibiting a sufficient flexibility. The composition of 2-hydroxybutyric acid is uniquely determined as the ratio between the homopolymer segment and the copolymer segment is determined.

In the copolymer of the present invention, a copolymer comprising 2-hydroxyacetic acid and 3-hydroxybutyrate has a homopolymer segment composed of 3-hydroxybutyrate, and a copolymer segment containing 2-hydroxyacetic acid and 3-hydroxybutyrate. The copolymer segment in this copolymer takes a random copolymer structure.

The molecular weight (number average molecular weight) of this copolymer is not particularly limited, but is preferably 10,000 or more, and more preferably 1,000,000 or more, from the viewpoint of exhibiting a mechanical strength and further keeping a sufficient molecular weight even after the molecular weight is reduced by melt molding when the copolymer is used as a material.

In addition, the content ratio between 2-hydroxyacetic acid and 3-hydroxybutyrate is not particularly limited, but when used as a hard segment, the content of 2-hydroxyacetic acid is preferably 50 mol% or more, and further preferably 70 mol% or more, from the viewpoint of controlling the mechanical physical properties of the material. On the other hand, when used as a soft segment, the content of 2-hydroxyacetic acid is preferably 50 mol% or less, and further preferably 10 to 30 mol%.

In the copolymer of the present invention, a copolymer comprising 2-hydroxyacetic acid, 3-hydroxybutyrate, and 3-hydroxyhexanoic acid has a homopolymer segment composed of 3-hydroxybutyrate, and a copolymer segment containing 2-hydroxyacetic acid, 3-hydroxybutyrate, and 3-hydroxyhexanoic acid. The copolymer segment in this copolymer takes a gradient polymer structure.

For the molecular weight (number average molecular weight) of this copolymer, the lower limit is not particularly limited, but is preferably 10,000 or more, and more preferably 1,000,000 or more when the copolymer is used as a material, from the viewpoint of exhibiting mechanical strength and further keeping a sufficient molecular weight even after the molecular weight is reduced by melt molding.

In addition, the content of 3-hydroxyhexanoic acid is preferably 10 mol% or more, and more preferably 30 mol% or more, of the entire content, from the viewpoint that the material exhibits flexibility. In this case, the contents of 2-hydroxyacetic acid and 3-hydroxybutyrate are both preferably 70 mol% or less, and both further preferably 50 mol% or less.

Similarly, the content of the hard homopolymer segment is preferably 50 mol% or less, and further preferably 30 mol% or less, from the viewpoint of providing the material with flexibility.

In the copolymer of the present invention, a copolymer comprising 2-hydroxybutyric acid, 3-hydroxybutyrate, and 3-hydroxypropionic acid, 4-hydroxy-2-methylbutyric acid, 2,4-dihydroxybutyric acid, 5-hydroxyvaleric acid, or 6-hydroxyhexanoic acid has a homopolymer segment composed of 2-hydroxybutyric acid and a copolymer segment containing 3-hydroxybutyrate and 3-hydroxypropionic acid, or the like. Note that the copolymer segment in this copolymer takes a random copolymer structure.

The molecular weight of this copolymer is preferably 100,000 or more, and more preferably around 1,000,000 (for example, 500,000 to 2,000,000, or 800,000 to 1,500,000) in terms of weight average molecular weight, for expressing strength that allows the copolymer to be processed into a material.

Although the composition of monomers of the random segment can be adjusted by a person skilled in the art in order to obtain target physical properties, in the case of using 3-hydroxybutyrate as a main component and synthesizing a random segment containing 3-hydroxypropionic acid, 4-hydroxy-2-methylbutyric acid, and 5-hydroxyvaleric acid or 6-hydroxyhexanoic acid, the components other than 3-hydroxybutyrate are preferably 0 to 50 mol% for the purpose of softening the poly-3-hydroxybutyrate skeleton, and is more preferable around 5 to 20 mol% (for example, 1 to 40 mol%, or 3 to 30 mol%) for expressing appropriate flexibility and strength. In the case of synthesizing a segment containing 2,4-dihydroxybutyric acid for the purpose of hydrophilization of a polymer, the composition of 2,4-dihydroxybutyric acid is preferably around 5 to 20 mol% (for example, 1 to 40 mol%, or 3 to 30 mol%). In the case of aiming at forming a soft segment using a unit (3-hydroxypropionic acid, 4-hydroxy-2-methylbutyric acid, 5-hydroxyvaleric acid, or 6-hydroxyhexanoic acid) other than 3-hydroxybutyrate as a main component, it is preferable to adjust the components other than 3-hydroxybutyrate to 50 to 100 mol%. In order to exhibit a soft physical property, the proportion of the components other than 3-hydroxybutyrate is preferably high, but is preferably around 80 to 95 mol% (for example, 60 to 99 mol%, or 70 to 97 mol%) in order to achieve a better balance with the efficiency of synthesizing the polymer.

In the copolymer of the present invention, a copolymer comprising 2-hydroxybutyric acid, 3-hydroxypropionic acid, 4-hydroxy-2-methylbutyric acid, and 2,4-dihydroxybutyric acid, 5-hydroxyvaleric acid, or 6-hydroxyhexanoic acid has a homopolymer segment composed of 2-hydroxybutyric acid, and a copolymer segment containing 3-hydroxypropionic acid and 4-hydroxy-2-methylbutyric acid, or the like. Note that the copolymer segment in this copolymer takes a random copolymer structure.

In this copolymer, the fractions of 3-hydroxypropionic acid and the other units in the segment can be used in any composition, and an example includes 100% or 90 mol% 3-hydroxypropionic acid. In addition, the molecular weight is preferably 100,000 or more in terms of the weight average molecular weight.

In the copolymer of the present invention, a copolymer comprising 2-hydroxybutyric acid, 3-hydroxybutyrate, and 3-hydroxyvaleric acid has a homopolymer segment composed of 2-hydroxybutyric acid, and a copolymer segment containing 3-hydroxybutyric acid and 3-hydroxybutyrate. Note that the copolymer segment in this copolymer takes a random copolymer structure.

In this copolymer, the copolymer segment preferably contains 10 to 30 mol% 3-hydroxyvaleric acid. The molecular weight is preferably 100,000 or more in terms of the weight average molecular weight.

### <Method for producing a block copolymer>

As shown in Examples described below, the above-mentioned copolymers of the present invention can be obtained by adding CoA to the above-mentioned hydroxycarboxylic acids through CoA transferase in a microorganism such as Escherichia coli, and further polymerizing these hydroxycarboxylic acids with a polymerizing enzyme. Hence, the present invention provides a method comprising the steps of:
Step 1: culturing a microorganism expressing a CoA transferase and a polymerizing enzyme in a medium containing a hydroxycarboxylic acid (A) and a hydroxycarboxylic acid (B); and
Step 2: collecting the copolymer from a culture obtained in the culturing step.

### (CoA Transferase)

The "CoA transferase" in the present invention means an enzyme that catalyzes reaction in which CoA (coenzyme A) is transferred to the above-mentioned hydroxycarboxylic acids. For example, in the case of targeting a hydroxycarboxylic acid having 5 or less carbon atoms, propionate CoA-transferase may be used.

The propionate CoA-transferase (PCT) means an enzyme classified into Enzyme Commission (EC) No.: 2.8.3.1. The PCT according to the present invention is not particularly limited in terms of its origin as long as the PCT keeps its catalytic activity. However, those derived from bacteria belonging to the genus Megasphera, the genus Ralstonia, the genus Pseudomonas, or the genus Escherichia are preferable, those derived from bacteria belonging to the genus Megasphera are more preferable, and that derived from Megasphaera elsdenii (a protein consisting of an amino acid sequence set forth in SEQ ID NO: 1) is further preferable, from the viewpoint that the heterologous expression in Escherichia coli is easy.

In addition, mutations of nucleotide sequences could occur also in the natural world. In association with this, amino acids to be encoded could also change. Hence, the PCT according to the present invention includes proteins (modifications) consisting of amino acid sequences obtained by substituting, deleting, adding, and/or inserting one or a plurality of amino acids in wild-type amino acid sequences derived from the above-mentioned bacteria (for example, the amino acid sequence set forth in SEQ ID NO: 1) as long as they have the above catalytic activity.

In the PCT according to the present invention, the term "plurality" means within 100 amino acids, preferably within 80 amino acids, more preferably within 50 amino acids, further preferably within 30 amino acids, more preferably 20 within amino acids, further preferably within 10 amino acids, and particularly preferably within several amino acids (for example, within 5 amino acids, within 3 amino acids, or within 2 amino acids).

Moreover, in the current state-of-the-art, it is possible for a person skilled in the art to identify the homologous genes from the same or other bacteria by utilizing information on the genes (nucleotide sequences). The methods for identifying homologous genes include, for example, the hybridization technique (Southern, E. M., J. Mol. Biol., 98: 503, 1975) and the polymerase chain reaction (PCR) technique (Saiki, R. K., et al. Science, 230: 1350-1354, 1985, Saiki, R.K. et al. Science, 239: 487-491, 1988). In order to identify a homologous gene, hybridization reaction is normally conducted under stringent conditions. As the stringent conditions for hybridization, conditions of 6M urea, 0.4% SDS, and 0.5xSSC or stringent conditions for hybridization similar to those can be exemplified. By using more stringent conditions, for example, conditions of 6M urea, 0.4% SDS, and 0.1xSSC, isolation of genes having a higher homology can be expected.

Hence, as long as the PCT has the catalytic activity, the PCT according to the present invention includes proteins encoded by DNAs that are composed of the wild-type nucleotide acid sequences derived from the bacteria and DNAs that hybridize under stringent conditions.

In addition, the protein encoded by the identified homologous gene normally has a high homology with (high similarity to) that derived from the bacterium, and preferably a high identity. Here, the term "high" means at least 50% or more, preferably 60% or more, more preferably 70% or more, further preferably 80% or more, and more preferably 85% or more (for example, 90% or more, 95% or more, 96% or more, 97% or more, 98% or more, or 99% or more) .

Hence, as long as the PCT has the catalytic activity, the PCT according to the present invention also includes proteins consisting of amino acid sequences having a homology (similarity) or an identity of at least 50% or more with or to wild-type amino acid sequences derived from the above-mentioned bacteria.

Note that the sequence homology can be determined by utilizing a program of BLAST (Altschul et al. J. Mol. Biol., 215: 403-410, 1990). The program is based on algorithm BLASTs (Proc. Natl. Acad. Sci. USA, 87: 2264-2268, 1990, Proc. Natl. Acad. Sci. USA, 90: 5873-5877, 1993) of Karlin and Altschul. For example, in the case of analyzing an amino acid sequence using BLAST, parameters are set to, for example, score=50 and wordlength=3. In addition, in the case of analyzing an amino acid sequence using Gapped BLAST program, the analysis can be conducted as described in Altschul et al. (Nucleic Acids Res. 25: 3389-3402, 1997). In the case of using BLAST and Gapped BLAST program, default parameters of each program are used. The specific procedures of these analyzing methods are publicly known.

It is possible for a person skilled in the art to determine whether or not the above-mentioned modification or homolog of the PCT has the catalytic activity, by using the method described in, for example, A. E. Hofmeister et al. (Eur. J. Biochem., vol. 206, 547-552) to measure the catalytic activity of these.

In addition, for example, in the case of targeting a hydroxycarboxylic acid having a hydroxy group at the 3-position, such as 3HHx, besides the aforementioned PCTs, acyl-CoA synthetase can be used as a favorable example of the CoA transferase according to the present invention.

The acyl-CoA synthetase (AlkK) means an enzyme classified into Enzyme Commission (EC) No.: 6.2.1.3. The AlkK according to the present invention is not particularly limited in terms of its origin as long as the AlkK keeps its catalytic activity. However, those derived from bacteria belonging to the genus Pseudomonas are preferable, those derived from Pseudomonas oleovorans or Pseudomonas putida are more preferable, those derived from Pseudomonas putida are further preferable, and that derived from Pseudomonas putida KT2440 (a protein consisting of an amino acid sequence set forth in SEQ ID NO: 2) is more preferable (see Wang et al. Appl Environ Microbiol. 2012 Jan; 78(2): 519-527), from the viewpoint that they have sufficient activities to medium-chain-length 3-hydroxycarboxylic acid.

In addition, like the PCT, the AlkK according to the present invention includes proteins (modifications) consisting of amino acid sequences obtained by substituting, deleting, adding, and/or inserting one or a plurality of amino acids in wild-type amino acid sequences derived from the above-mentioned bacteria (for example, the amino acid sequence set forth in SEQ ID NO: 2) as long as they have the above catalytic activity. In the AlkK according to the present invention, the term "plurality" means within 100 amino acids, preferably within 80 amino acids, more preferably within 50 amino acids, further preferably within 30 amino acids, more preferably within 20 amino acids, further preferably within 10 amino acids, particularly preferably within several amino acids (for example, within 5 amino acids, within 3 amino acids, or within 2 amino acids).

Moreover, the AlkK according to the present invention also includes proteins encoded by DNAs that hybridize with DNAs composed of wild-type nucleotide acid sequences derived from the above-mentioned bacteria under stringent conditions and proteins consisting of amino acid sequences having a homology (similarity) or an identity of at least 50% or more with or to wild-type amino acid sequences derived from the above-mentioned bacteria, as long as they have the catalytic activity.

In addition, it is possible for a person skilled in the art to determine whether or not the modification or homolog of the AlkK has the catalytic activity, for example, by using a method that specifically oxidizes acyl-CoA generated by reaction of the enzyme by using acyl-CoA oxidase, causes the hydrogen peroxide thus generated to produce color by using 4-aminoantipyrine, and 3-methyl-N-ethyl-N-(2-hydroxyethyl)aniline, N,N-dimethylaniline, N,N-diethylaniline, or phenol in the presence of a peroxidase, and detects the color production.

### (Polymerizing Enzyme)

In the present invention, the "polymerizing enzyme" only has to be an enzyme that catalyzes reaction of polymerizing the hydroxycarboxylic acid to which the aforementioned CoA has been transferred to form the above-mentioned homopolymer segment and copolymer segment, and includes, for example, polymerizing enzymes that can recognize (1) a hydroxycarboxylic acid having a hydroxy group only at the 2-position and (2) a hydroxycarboxylic acid having no hydroxy group at the 2-position to control the polymerization reaction. The polymerizing enzyme is preferably type 1 polyhydroxyalkanoate synthase (PhaC) or a modification thereof having the catalytic activity, and is further preferably type 1 PhaC or a modification thereof derived from bacteria belonging to the genus Aeromonas, and is more preferably type 1 PhaC or a modification thereof derived from Aeromonas caviae. The polymerizing enzyme is further preferably a chimeric polymerizing enzyme obtained by fusing the N-terminal portion of type 1 PhaC derived from bacteria belonging to the genus Aeromonas and the C-terminal portion of type 1 PhaC derived from bacteria belonging to the genus Ralstonia, is more preferably a chimeric polymerizing enzyme obtained by fusing the N-terminal portion of type 1 PhaC derived from Aeromonas caviae and the C-terminal portion of type 1 PhaC derived from Ralstonia eutropha, and is further preferably a chimeric polymerizing enzyme obtained by fusing portion of 26% on the N-terminal side of type 1 PhaC derived from Aeromonas caviae and portion of 74% on the C-terminal side of type 1 PhaC derived from Ralstonia eutropha (PhaC_{AR}, (a protein consisting of the amino acid sequence set forth in SEQ ID NO: 3)) (see Matsumoto K. et al., Biomacromolecules. 2009 Apr 13; 10(4): 682-685).

In addition, like the above-mentioned CoA transferase, the polymerizing enzyme according to the present invention includes proteins (modifications) consisting of amino acid sequences obtained by substituting, deleting, adding, and/or inserting one or a plurality of amino acids in the amino acid sequence of the enzyme (for example, the amino acid sequence set forth in SEQ ID NO: 3) as long as they have the catalytic activity. In the CoA transferase according to the present invention, the term "plurality" means within 100 amino acids, preferably within 80 amino acids, more preferably within 50 amino acids, further preferably within 30 amino acids, more preferably within 20 amino acids, further preferably within 10 amino acids, particularly preferably within several amino acids (for example, within 5 amino acids, within 3 amino acids, or within 2 amino acids).

Such modification includes, for example, substitution of at least one amino acid selected from the group consisting of F314X, I320X, G423X, V448X, and I505X in PhaC_{AR} ("X" means an amino acid different from that before the substitution). Since such substitution of amino acids enhances the 3HHx polymerizability, in the case where 3HHx is used as the hydroxycarboxylic acid (B), for example, it becomes possible to improve the 3HHx fraction in the copolymer according to the present invention.

Furthermore, the polymerizing enzyme according to the present invention includes proteins encoded by DNAs that hybridize with DNAs composed of nucleotide acid sequences encoding the enzymes under stringent conditions and proteins consisting of amino acid sequences having a homology (similarity) or an identity of at least 50% or more with the amino acid sequences of the enzymes as long as they have the catalytic activity.

In addition, it is possible for a person skilled in the art to determine whether or not the modification or homolog of the polymerizing enzyme has the catalytic activity, for example, by using the method described in NPL 2.

### (Microorganism)

In the present invention, the copolymer of the present invention can be produced by culturing a microorganism expressing the above-mentioned two types of enzymes. As such a microorganism, those expressing these enzymes in nature can be used, but transformants prepared by introducing DNAs encoding the enzymes into a microorganism are normally used.

The "microorganism" according to the present invention is not particularly limited as long as the microorganism can functionally express the polyhydroxyalkanoate (PHA) synthase group, but the microorganism includes, for example, Escherichia coli, hydrogen oxidizing bacteria (Cupriavidus necator), coryneform bacteria, yeast, Ralstonia bacteria, and Pseudomonas bacteria. Among these microorganisms, Escherichia coli and hydrogen oxidizing bacteria are preferable, and Escherichia coli is more preferable, from the viewpoint of the polymer productivity and the production stability. The strain of Escherichia coli is not particularly limited, but includes E. coli JM109 strain as an example from the viewpoint that more stable production of copolymers becomes possible. In addition, as shown in Examples described later, from the viewpoint that the hydroxycarboxylic acid (B) (3HB, 3HV, 3HHx, or the like) according to the present invention can be biosynthesized, Ralstonia bacteria are preferable, Ralstonia eutropha is more preferable, the Ralstonia eutropha glucose-utilizing modified strain (H16 NSDG-GG-ΔB1 strain) is further preferable, and H16 CAR-GG-ΔB1, and H16 CAR-GG-ΔB1-ldhA hadA shown in Examples described later are more preferable.

In such a microorganism, in order to express two types of enzymes according to the present invention, the above DNAs are normally introduced in the form of being incorporated in vector. Alternatively, the DNAs may be introduced into chromosomes through homologous recombination. Note that in order for the DNA (gene) introduced into chromosomes of the microorganism according to the present invention to be appropriately transferred and further translated to proteins having desired activity, the DNA needs to be incorporated in such a manner as to be under the control of an appropriate promoter on the chromosome.

In the present invention, the "vector" can be constructed, for example, based on a plasmid which exists as an autonomously replicating vector, that is, an extrachromosomal element and can replicate independently of the replication of chromosomes. Alternatively, the vector may be a vector which is integrated into the genome of a host cell when introduced into the host cell and replicated together with the chromosomes in which the vector is incorporated.

Such a vector includes, for example, plasmid DNAs and phage DNAs. Examples of vectors for introduction into Escherichia coli include plasmid DNAs such as pBLuescripts (pBLuescript KS+, pBLuescript SK+, pBLuescriptII KS+, pBLuescriptII KS-, and the like), pBR322, and pUC18 and phage DNAs such as EMBL3, M13, and AgtII. Examples of vectors for introduction into hydrogen oxidizing bacteria include pCUP2 (see International Publication No. WO2007/049716), pLA2917 (ATCC37355) having a RK2 replication origin which may be replicated and retained by a host of range, and pJRD215 (ATCC37533) having RS F1010 replication origin. Examples of vectors for introduction into coryneform bacteria include pAM330, pH M1519, pAJ655, pAJ611, pAJ1844, pCG1, pCG2, pCG4, pCG11, pH K, pPSPTG1, pVC7, pUC19I, and the like. Examples of vectors for introduction into yeast include YEp13, YCp50, and the like. In addition, examples of vectors for introduction into Ralstonia bacteria, Pseudomonas bacteria, and the like include the aforementioned pLA2917 (ATCC37355) and pJRD215(ATCC37533). In addition, vectors for homologous recombination include, for example, pK18mobsacB (Schafer et al., Gene 145, 69-73 (1994)), pJQ200 (Quandt, J. and Hynes, M. P., Gene (1993) 17: 15-21).

For the insertion of DNA encoding each of the above-mentioned enzymes into a vector, a publicly-known gene-recombination technology conventionally used in the field of genetic engineering can be used. For example, in order to insert the DNA into a vector, a method that cleaves the purified DNA by using an appropriate restriction enzyme, and inserts the cleaved DNA into a restriction enzyme site or multiple cloning site of an appropriate vector and binds the DNA to the vector, and the like can be employed.

It is preferable in recombination to bind the DNA downstream of a promoter that can adjust the transcription and translation of each protein from the DNA introduced into a vector. The promoter is not particularly limited as long as the promoter can adjust the transcription of gene in a host, and can be obtained as a DNA sequence that controls expression of a gene encoding a protein the same as or different from the host cell. For example, in the case where Escherichia coli is used as a host, the lac promoter, the trp promoter, the PL promoter, the PR promoter, and the T7 promoter can be used. Alternatively, the promoter region of the phb operon of Cupriavidus necator can also used. In the case where hydrogen oxidizing bacteria are used as a host, the phaC1 gene promoter derived from this bacterium and the phaP1 gene promoter derived from this bacterium can be used. In the case where coryneform bacteria are used as a host, for example, the cell-surface protein gene promoter derived from Corynebacterium glutamicum can be used. In the case where yeast is used as a host, for example, the gall promoter and the gal10 promoter can be used. In the case where Pseudomonas bacteria are used as the microorganism of the present invention, a region containing a promoter located upstream of the phaC1Ps gene or upstream of the phbCABRe operon can be used.

The vector also contains a sequence that controls expression other than the promoter, such as a terminator sequence, an enhancer sequence, a splicing signal sequence, a poly-A additional signal sequence, or a ribosome-binding sequence (SD sequence) that can be used in the microorganism into which the DNA is to be introduced, as necessary. In addition, the vector may contain a sequence that induces expression other than the sequence. Such sequence includes the lactose operon that can induce expression of a gene arranged downstream by adding isopropyl β-d-1-thiogalactopyranoside (IPTG).

The vector may contain a selective marker gene as necessary. The selective marker gene may be selected as appropriate depending on the method for selecting a transformed microorganism cell, and includes, for example, drug resistance genes such as the ampicillin resistance gene, the tetracycline resistance gene, the neomycin resistance gene, the kanamycin resistance gene, and the chloramphenicol resistance gene, genes involved in intracellular biosynthesis of nutrients (genes complementary to auxotrophy) such as amino acids and nucleic acids, gene involved in chemiluminescence such as luciferase, and genes encoding fluorescent proteins such as GFP.

One type of the DNAs encoding the enzymes (CoA transferase and polymerizing enzyme) according to the present invention may be inserted into each vector, or a plurality of types of the DNA may be inserted into one vector. In the case where a plurality of types of DNAs are inserted into the vector, when the plurality of types of DNAs are inserted into one vector, it is preferable that these DNAs form an operon. Here, the "operon" is a nucleic acid sequence unit composed of one or a plurality of genes to be transcribed under control of the same promoter.

The above DNAs, or the vector into which the DNAs have been inserted, are introduced into a microorganism by using a publicly-known method. The method for introducing DNAs into a microorganism include, for example, the calcium chloride method, the heat shock method, the calcium phosphate method, the electroporation method, the spheroplast method, the lithium acetate method, the conjugal transfer method, and a method using calcium ions.

### (Culture Conditions for Medium or the Like)

In the present invention, the copolymer of the present invention can be produced by culturing the aforementioned microorganism in a medium containing a hydroxycarboxylic acid (A) and a hydroxycarboxylic acid (B).

The medium is not particularly limited as long as the medium is supplied with a carbon source of a sufficient concentration. The medium may contain a nitrogen source which is a nutrient source other than the carbon source, an inorganic salt, or another organic nutrient source.

As the carbon source, any type of carbon source may be used, as long as the above-mentioned microorganism can assimilate the carbon source, but preferable carbon sources include sugars such as glucose, fructose, and sucrose; oils and/or fats such as palm oil, palm kernel oil, corn oil, coconut oil, olive oil, soybean oil, rapeseed oil, and Jatropha oil and fractionated oils thereof; fatty acids such as lauric acid, oleic acid, stearic acid, palmitic acid, and myristic acid and derivatives thereof.

The nitrogen source include, for example, ammonia; ammonium salts such as ammonium chloride, ammonium sulfate, and ammonium phosphate; peptone, meat extract, and yeast extract. The inorganic salts include, for example, monobasic potassium phosphate, disodium phosphate, phosphoric acid magnesium, magnesium sulfate, and sodium chloride. The other organic nutrient sources include, for example, amino acids such as glycine, alanine, serine, threonine, and proline; and vitamins such as vitamin B1, vitamin B12, and vitamin C.

Examples of the medium according to the present invention include an LB medium (containing 10 g/L tryptone, 5 g/L yeast extract, and 5 g/L sodium chloride) in the case of culturing Escherichia coli or the like, but is not limited to these. In addition, in the case of using natural PHA-producing bacteria (for example, hydrogen oxidizing bacteria, and Pseudomonas putida) as a host, the medium includes a nitrogen-limited, inorganic salt medium (containing, for example, 9 g/L Na₂HPO₄, 1.5 g/L KH₂PO₄, 0.5 g/L NH₄Cl, 0.2 mg/L MgSO₄, and trace elements), and a phosphorus-limited, inorganic salt medium (containing, for example, 1 g/L glucose, 0.5 g/L KNO₃, 0.2 g/L MgSO₄(7H₂0), 0.1 g/L CaC1₂, 0.1 g/L NaC1, to which 8.0 mg of K₂HPO₄ and 2.0 mg of KH₂P0₄ have been added, and in which pH has been adjusted to 7), but is not limited to these.

The hydroxycarboxylic acids to be added to the medium is as described above, and may be R-form or racemate, and is preferably R-form. In addition, the hydroxycarboxylic acids may be added after the pH is adjusted (neutralized or the like) in accordance with the optimum pH of the medium or may be added in the form of a salt. Such a salt includes sodium salt, potassium salt, ammonium salt, and the like. The amount of the hydroxycarboxylic acids to be added to the medium only has to be at a concentration suitable for copolymerization synthesis, and may be adjusted as appropriate to control the composition of monomers during copolymerization, which is synthesized depending on the concentration, and is, for example, 1 to 10 g/L.

In addition, as shown in Examples described later, even when the hydroxycarboxylic acid (A) and the hydroxycarboxylic acid (B) are not added to the medium, if the microorganism is capable of biosynthesizing these hydroxycarboxylic acids, it is possible to produce the copolymer of the present invention by using these metabolic precursors (starting materials of the metabolism, or the like).

Hence, the present invention can be rephrased to a method comprising the steps of:
culturing a microorganism expressing a CoA transferase and a polymerizing enzyme in a medium containing a hydroxycarboxylic acid (A) and/or a metabolic precursor thereof and a hydroxycarboxylic acid (B) and/or a metabolic precursor thereof; and
Step 2: collecting the copolymer from a culture obtained in the culturing step.

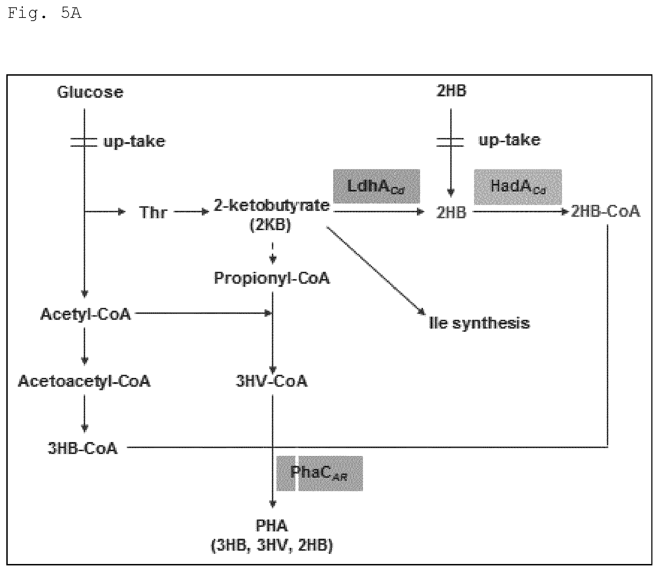

The metabolic precursors according to the present invention are not particularly limited as long as they are substances that are eventually converted to the hydroxycarboxylic acids according to the present invention through metabolic pathways in the microorganism. For example, in the case where the hydroxycarboxylic acid (A) is 2HB, the metabolic precursor may be glucose, threonine, 2-ketobutyric acid, or the like. For example, in the case where the hydroxycarboxylic acid (B) is 3HB, the metabolic precursor may be glucose, pyruvic acid, acetyl CoA, acetoacetyl-CoA, or the like. For example, in the case where the hydroxycarboxylic acid (B) is 3HV, the metabolic precursor may be 2HB, 2-oxobutyric acid, threonine, propionic acid, propionic acid-IP, propionic acid-CoA, 3-ketovaleryl-CoA, or the like (see Fig. 5A and the like).

As the conditions for culturing the above-mentioned microorganism, besides the aforementioned medium, the culture temperature is not particularly limited as long as the culture temperature is within a range in which the microorganism used can grow and each enzyme activity can be exhibited, and is normally 25 to 37°C, preferably 28 to 32°C, and particularly preferably 30°C. In addition, which one of an aerobic condition and an anaerobic condition is employed can be selected as appropriate depending on the type of the microorganism used, but the aerobic condition is preferable from the viewpoint that the synthesis of the copolymer is more likely to be accelerated. The culture time can be adjusted as appropriate depending on the amount of the copolymer produced, and the like, but is normally 12 hours to 1 week, and preferably 1 to 3 days.

### (Collection of the Copolymer and the like)

In the present invention, the copolymer of the present invention is collected from the culture obtained by culturing the above-mentioned microorganism. Note that the "culture" includes not only the cultivated microorganism obtained by culturing the microorganism in the medium, but also the medium containing the secretion products and metabolites, and the like of the microorganism, as well as dilutions and concentrates of these.

The method for collecting the copolymer from such a culture is not particularly limited, but the following method can be conducted, for example. After completion of the culturing, the microorganism is separated from the medium by means of centrifugation processing or the like, and the microorganism is washed by using distilled water and methanol or the like and dried (for example, freeze dried). These dried bacterial cells are subjected to heating treatment using an organic solvent such as chloroform to extract the copolymer. From the solution of the organic solvent containing the copolymer, bacterial cell components are removed by using filtration or the like, and a poor solvent such as methanol or hexane is added to the filtrate to cause the copolymer to precipitate. Moreover, a supernatant liquid was removed by means of filtration or centrifugation to dry and collect the copolymer.

In addition, for the copolymer obtained in this way, the average molecular weight, the composition of monomers, the structure, and the like can be checked by using publicly-known analytical methods such as the nuclear magnetic resonance (NMR), gel permeation chromatography, gas chromatography, or the like as shown in Examples described later.

### [Examples]

Hereinafter, the present invention will be described in further detail based on Examples, but the present invention is not limited to the following Examples. In addition, Examples were conducted by using methods described below.

### (Example 1)

### <Method for preparing P(2HB-b-(3HB-ran-3HHx))>

A plasmid (pBSPreCARpctAlkK) in which the promoter region of the phb operon of C.nector, the PhaC_{AR} gene, the pct gene derived from Megasphaera elsdenii, and the AlkK gene derived from Pseudomonas putida KT2440 were inserted in this order into pBluescript KS+plasmid was prepared. Note that the plasmid was prepared by inserting the AlkK gene downstream of the pct gene in pBSP_{Re}phaC(AR)pct (see NPL 2) by using a conventional method. Then, this plasmid was introduced into Escherichia coli (JM109 strain) by using the calcium chloride method to obtain a transformant.

This transformants were shake-cultured at 30°C for 48 hours in LB media containing 1 to 10 g/mL (R,S)-2HB-Na, 1 to 10 g/mL (R,S)-3HB-Na, and 1 to 10 g/mL (R,S)-3HHx-Na.

Escherichia coli collected from the obtained culture solution was freeze-dried and heated with chloroform to collect the polymer (see the collecting method described in Taguchi,S. et al., Proc. Natl. Acad. Sci. U.S A. 2008, 105(45), 17323-17327).

### <Analysis on the structure of P(2HB-b-(3HB-ran-3HHx))>

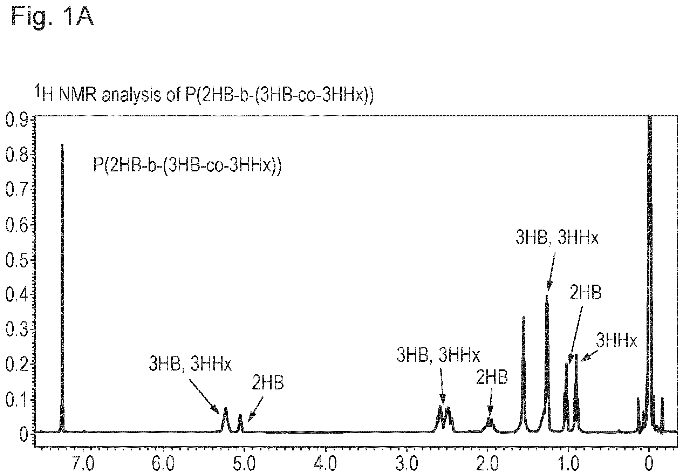

(R,S)-2HB-Na, (R,S)-3HB-Na, and (R,S)-3HHx-Na were added to the LB medium each in an amount of 2.5 g/mL, and the obtained polymer was dissolved in deuterochloroform, followed by conducting ¹H NMR measurement to calculate the composition and the mol fraction. As a result, as shown in Fig. 1A, since only one 5 ppm signal derived from 2HB was observed, it was revealed that the P(2HB) segment was present.

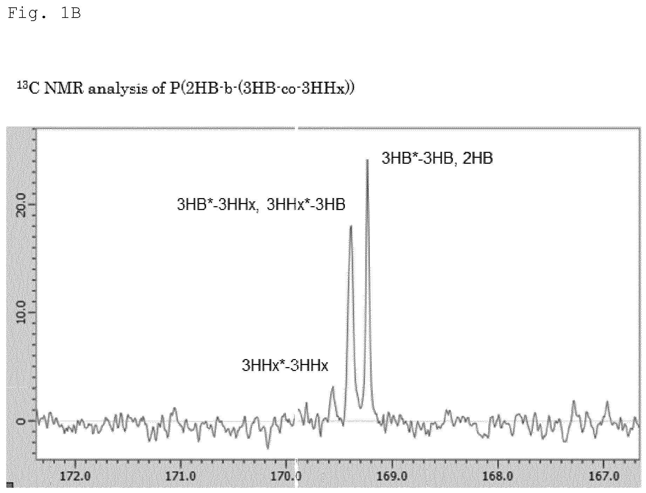

In addition, the same sample was further subjected to ¹³C NMR measurement. As a result, as shown in Fig. 1B, since the chain strength between 3HHx and 3HHx, it was found that no P(3HHx) homopolymer segment was present, that is, the structure containing 3HHx units was a random sequence.

From the above, it was found that the polymer obtained in the above was a block copolymer composed of P(2HB) homopolymer segments and P(3HB-ran-3HHx) random segments.

### <Analysis on the physical properties of P(2HB-b-(3HB-ran-3HHx))>

Around 100 mg of the polymer was dissolved in chloroform and the solvent was vaporized in a glass petri dish to form a solvent-cast film. From the circular film thus obtained, a test piece was cut out and subjected to a tensile test to calculate parameters such as the elongation at break (extensibility) and the tensile strength.

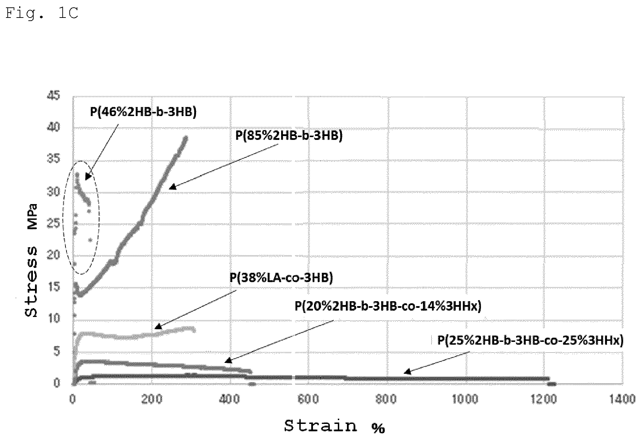

As is clear from the results shown in Fig. 1C, the ternary copolymer P(2HB-b-(3HB-ran-3HHx)) obtained at this time exhibited a higher extensibility than the binary copolymer P(2HB-b-3HB) disclosed in NPL 2. In particular, the ternary copolymer exhibited a significantly high extensibility of more than 1200%. From these results, it is also suggested the present invention makes it possible to obtain a copolymer exhibiting elastomeric properties in which strain and stress are proportional to each other by controlling the sequence of monomers, and thus controlling the high-order structure.

### (Example 2)

### <Method for preparing P(3HB-b-(GL-ran-3HB))>

Like Example 1, pBSPreCARpctAlkK was introduced into Escherichia coli (JM109 strain) by using the calcium chloride method to obtain a transformant.

In addition, a transformant in which a plasmid DNA encoding PhaC1_{Ps}STQK was introduced in place of the plasmid DNA encoding PhaC_{AR} was prepared. Note that this plasmid was fabricated by substituting the PhaC1_{Ps}STQK gene for the PhaC_{AR} gene in the pBSPreCARpctAlkK by a conventional method.

Then, these transformants were shake-cultured at 30°C for 48 hours in LB media containing 0 to 12 g/L (R,S)-GL-Na and 5 g/L (R,S)-3HB-Na, and polymers were collected from the culture solutions thus obtained in the same method as described in Example 1.

### <Analysis on P(3HB-b-(GL-ran-3HB))>

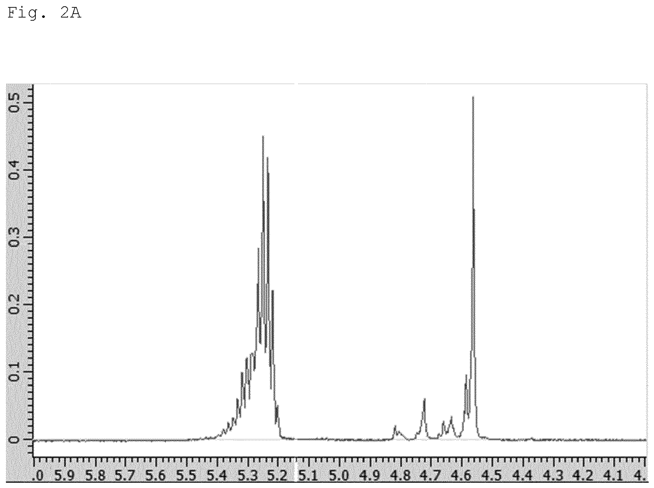

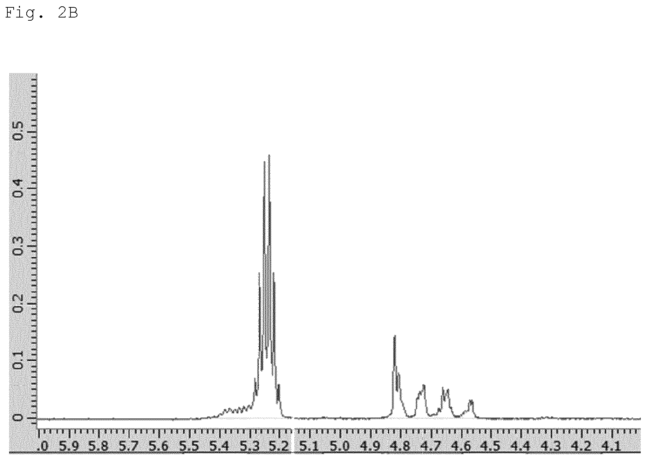

The bacterial cells after the culturing were freeze-dried and subjected to chloroform extraction to extract the polymer in the cells. The chloroform solution from which the bacterial cells were filtrated was concentrated, and then excess methanol was added to precipitate the polymer. The polymer collected was dissolved in deuterochloroform, followed by conducting ¹H NMR measurement. The obtained results are shown in Figs. 2A and 2B. Note that these figures show the results of analyzing the polymers obtained by using a LB medium containing 12 g/L (R,S)-GL-Na and 5 g/L (R,S)-3HB-Na. In addition, the compositions of monomers were calculated based on the peak intensities. The obtained results are shown in Table 1.

**[Table 1]**

| **Polymerizing enzyme** | **GL-Na [g/L]** | **CDW [g/L]** | **polymer [g/L]** | **mol%** | |
|---|---|---|---|---|---|
| | | | | **3HB** | **GL** |
| **PhaC1_{Ps}STQK** | **0** | **3.7±0.14** | **0.43±0.05** | **100±0** | **0±0** |
| | **4** | **3.3±0.04** | **0.39±0.03** | **89±0.8** | **11±0.8** |
| | **12** | **2.8±0.06** | **0.34±0.04** | **84±0.4** | **16±0.4** |
| **PhaC_{AR}** | **0** | **3.8±0.07** | **0.32±0.03** | **100±0** | **0±0** |
| | **4** | **3.6±0.08** | **0.27±0.03** | **81±1.2** | **19±1.2** |
| | **12** | **3.4±0.12** | **0.23±0.06** | **82±1.6** | **18±1.6** |

### <Gel filtration chromatography (GPC) analysis on P(3HB-b-(GL-ran-3HB))>

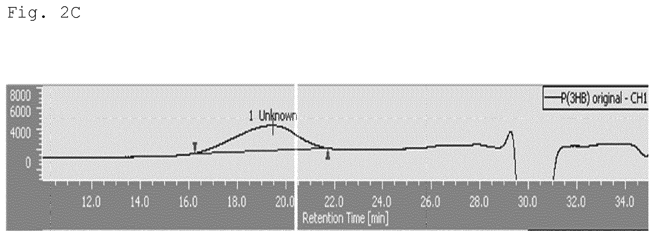

The bacterial cells after the culturing were freeze-dried and subjected to chloroform extraction to extract the polymer in the cell. In the analysis, a PTFE membrane was used for filtration, and the polymer was subjected to GPC analysis using chloroform as the mobile phase. The obtained result is shown in Fig. 2C. Note that this figure shows the result of analyzing the polymer obtained by using a LB medium containing 12 g/L (R,S)-GL-Na and 5 g/L (R,S)-3HB-Na.

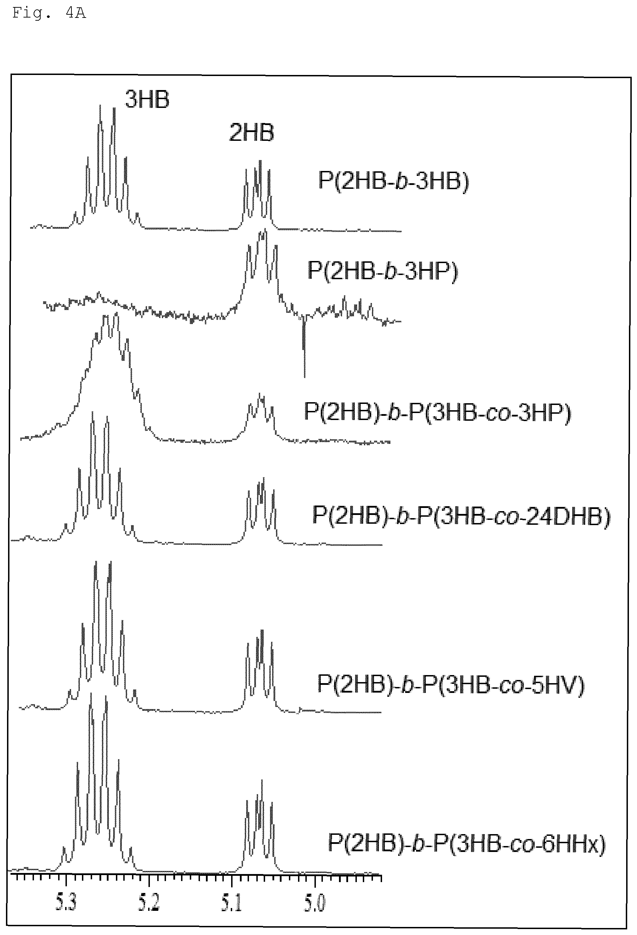

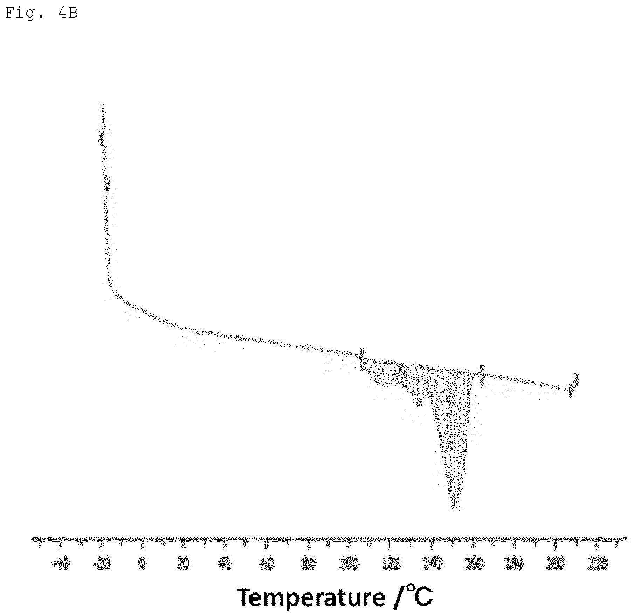

As a result of the above NMR analysis, in Figs. 2A and 2B, 4 peaks observed in 4.5 to 4.9 ppm are signals attributable to the GL units, and the peak on the lowest magnetic field side (left side) corresponds to the GL unit located between 3HBs (3HB-GL-3HB). In the case of a random copolymer having a GL fraction of around 20 mol%, it is expected that the signal of 3HB-GL-3HB is strongly observed.

From the above, it was determined that the polymer obtained using PhaC1_{Ps}STQK was a random copolymer (P(GL-ran-3HB)) (see Fig. 2A). On the other hand, for the polymer obtained using PhaC_{AR}, the signal of GL-GL-GL was strongly observed, and thus it was understood that the polymer contained a structure having a high GL density. From the relative intensity ratio of 4 peaks, the GL fraction was assumed to be about 70 mol%, which was greatly different from about 20 mol% of the GL fraction of the entire polymer. For this reason, it was revealed that this polymer had a random-block structure (P(3HB-b-(GL-ran-3HB))).

In addition, in the above gel filtration chromatography analysis, in the case where the polymer is a mixture of two or more types (for example, a copolymer with P(3HB)), multiple molecular weight distributions are observed. However, for the polymer obtained by using PhaC_{AR}, as shown in Fig. 2C, a unimodal peak was observed, which supported that the polymer had a sequence structure in the same molecule.

As described above, the polymer obtained by using PhaC_{AR} contains the P(3HB) homopolymer segment and the P(GL-ran-3HB) random copolymer segment, and has a sequence structure in which the compositions are greatly different in the same molecular chain, for which there has been no synthesis example at all in biosynthesized PHAs so far

Such a polymer mainly has two advantages. The first one is that since a structure in which a plurality of polymers having different physical properties are bound can be formed in the same molecule, it becomes possible to create a novel material attributable to the structure. The second one is that since the polymer contains many chains of GL units, the non-enzymatic hydrolyzability is improved (see Matsumoto K. et al., ACS Biomaterials Science & Engineering, 3(12) 3058, 2017).

### (Example 3)

### <Method for preparing P(3HB-b-(GL-co-3HB-co-3HHx))>

pBSPreCARpctAlkK was introduced into Escherichia coli (JM109 strain) by using the calcium chloride method to obtain a transformant. This transformant was shake-cultured at 30°C for 48 hours in an LB medium containing 2.5 to 5 g/L (R,S)-GL-Na, 0 to 2.5 g/L (R,S)-3HB-Na, and 1 g/L (R,S)-3HHx-Na, and a polymer was collected from the culture solution thus obtained in the same method as described in Example 1.

### <Analysis on P(3HB-b-(GL-co-3HB-co-3HHx))>

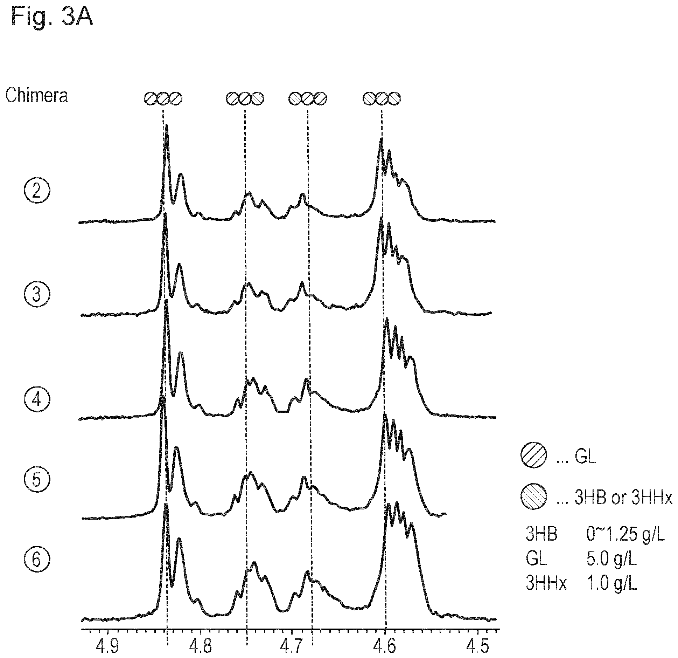

The ¹H NMR measurement was conducted in the same method as described in the above <Analysis on P(3HB-b-(GL-ran-3HB))>. The obtained result is shown in Fig. 3A. Note that this figure is a result of analyzing the polymer obtained by using an LB medium containing 5 g/L (R,S)-GL-Na, 2.5 g/L (R,S)-3HB-Na, and 1 g/L (R,S)-3HHx-Na, and the signal of the GL unit is enlarged. In addition, the composition of monomers was calculated from the peak area value. The obtained result is shown in Table 2.

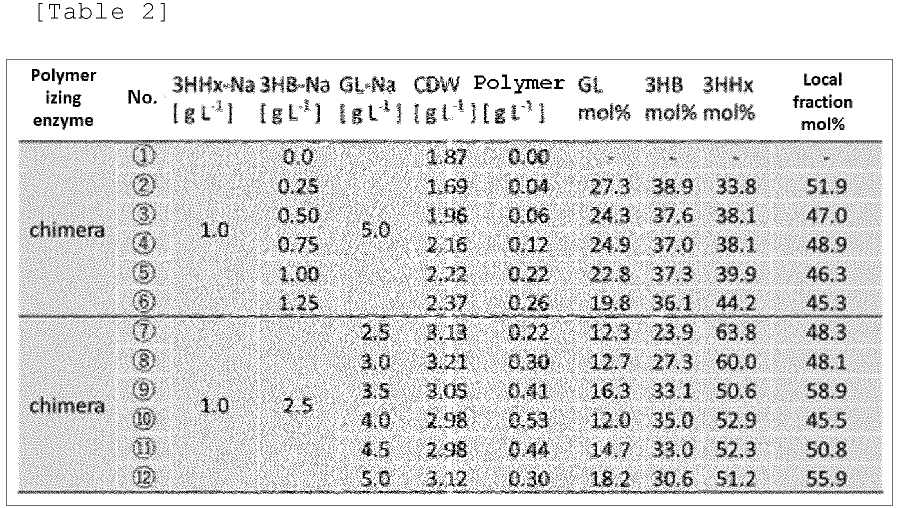

### <Differential scanning calorimetry (DSC) on P(3HB-b-(GL-co-3HB-co-3HHx))>

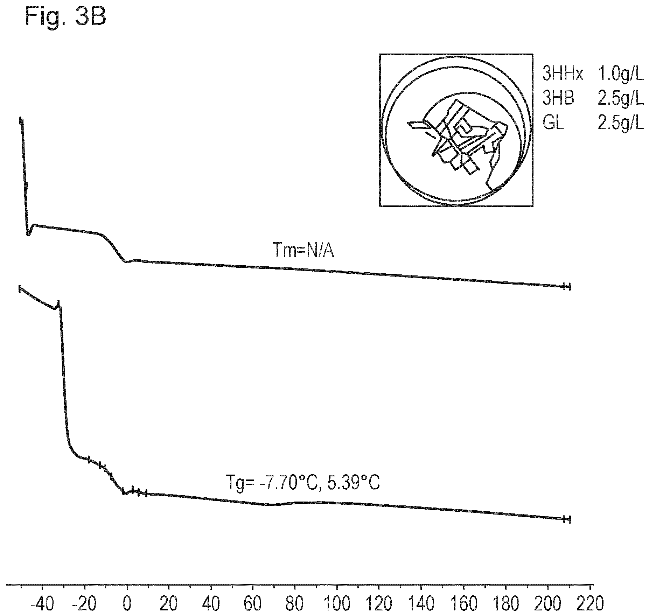

The polymer was dissolved in chloroform and the solvent was vaporized in a glass petri dish to form a solvent-cast film. A test piece of several milligrams was cut out from the obtained film and encapsulated in an aluminum pan to form a sample. This sample was subjected to DSC analysis. The DSC analysis was conducted such that the temperature was increased from -50°C to 220°C and whether or not there was an endothermic peak associated with the melting of the crystal was determined. The obtained results are shown in Fig. 3B.

As shown in Table 2, it was revealed that the present invention makes it possible to synthesize a ternary copolymer containing GL, 3HB, and 3HHx. In addition, in the ternary copolymer obtained in this way, 3HHx can be increased to 60 mol%. This is sufficient amount for the purpose of adding flexibility to the polymer properties. In addition, it is possible to increase the GL fraction to about 30 mol%, and it thus becomes possible to achieve a GL unit fraction sufficient for the purpose of modifying the material properties.

In addition, as shown in Fig. 3A, when the signal of the GL unit was increased in the above result of the NMR analysis, it was revealed that this polymer had a gradient structure based on the relative intensity of the observed 4 signals. Since in the gradient structure, both ends and the center portion of the molecule have different structures from each other, the molecule takes a structure similar to triblock.

Moreover, as shown in Fig. 3B, no crystal fusion peak was observed at all in the DSC analysis. This suggests that the present polymer takes a structure having a very low crystallinity.

From the above result, it was revealed that the present polymer contained an internal structure having a high GL fraction like P(3HB-b-(GL-ran-3HB)) shown in Example 2. On the other hand, it can be determined that 3HB units and 3HHx units are polymerized with each other at random. Hence, the present polymer has a unique structure (P(3HB-b-(GL-co-3HB-co-3HHx))), which is obtained by adding a GL-rich region to the conventional 3HB, 3HHx random copolymer.

### <Analysis on the physical properties of P(3HB-b-(GL-co-3HB-co-3HHx))>

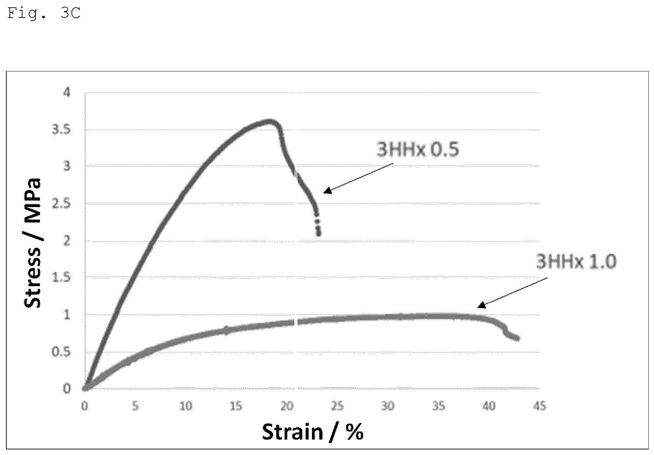

The polymer was dissolved in chloroform and the solvent was vaporized in a glass petri dish to form a solvent-cast film. From the film thus obtained, a test piece was cut out and subjected to the tensile test. The obtained results are shown in Fig. 3C.

Since the result of the tensile test on the P(3HB-b-(GL-co-3HB-co-3HHx)) as shown in Fig. 3C has a shallow gradient, it is understood that the polymer has a soft property. In addition, the polymer can be extended a little. Thus, it was revealed that it was possible to significantly improve the flexibility of the present polymer by introducing the soft 3HHx units.

In addition, with the GL units introduced, the present polymer has an increased non-enzymatic hydrolyzability, and, for example, is expected to have a high degradability in vivo and is useful in medical devices (for example, suture) and the like.

### (Example 4)

### <Method for Preparing a block copolymer comprising a P(2HB) homopolymer segment and a random copolymer segment mainly composed of 3-hydroxy acid, and analysis on the block copolymer>

As described in Example 1, when E. coli JM109 strain into which the plasmid pBSPreCARpctAlkK is introduced is cultured in the medium to which 2HB, 3HB, and 3HHx are added, a block copolymer composed of the P(2HB) homopolymer segment and the P(3HB-ran-3HHx) random segment is obtained.

In view of this, in the present Examples, the culturing (shake-culture at 30°C for 48 hours) was conducted in a LB medium to which 1.0 g/L a sodium salt of 3-hydroxypropionic acid (3HP), 4-hydroxy-2-methylbutyric acid (4H2MB), 2,4-dihydroxybutyric acid (24DHB), 5-hydroxyvaleric acid (5HV), or 6-hydroxyhexanoic acid (6HHx) was added instead of 3HHx in the culture system described in Example 1, and it was checked whether a copolymer containing each monomer unit was synthesized as in the case of Example 1. The obtained results are shown in Figs. 4A and 4B. Note that Fig. 4A shows results of conducting ¹H NMR measurement as in the method described in the above <Analysis on P(3HB-b-(GL-ran-3HB))>. Fig. 4B shows results of analysis like the above <Differential scanning calorimetry (DSC) on P(3HB-b-(GL-co-3HB-co-3HHx))>.

As shown in Fig. 4A, the presence of P(2HB) homopolymer segment was confirmed by the ¹H NMR measurement. To put it more specifically, since resonance attributable to the methine group of 2HB was observed near 5.1 ppm from all of the polymers, it was suggested that the P(2HB) segment was present.

In addition, it was confirmed from measurement of the melting point that the segment mainly composed of 3HB was a random copolymer. In the thermogram of the differential calorimetry of P(3HB-co-5HV), exemplified in Fig. 4B, the position of the peak representing the melting point is lower than around the melting point (178°C) of the P(3HB). From this, it can be confirmed that 5HV were randomly copolymerized. Note that although not illustrated, similar thermograms were obtained for the other copolymers.

From the above-described results, it was confirmed that the obtained polymers were such that the newly added monomer precursor was taken as a random copolymer with the 3HB unit and the 2HB component was polymerized as the P(2HB) homopolymer segment. More specifically, it was confirmed that it was possible to obtain novel ternary block copolymers, P(2HB)-b-P(3HB-ran-3HP), P(2HB)-b-P(3HB-ran-4H2MB), P(2HB)-b-P(3HB-ran-24DHB), P(2HB)-b-P(3HB-ran-5HV), and P(2HB)-b-P(3HB-ran-6HHx) under the above culture conditions.

Moreover, in the present invention, a block copolymer comprising a P(2HB) homopolymer segment and a random copolymer of a segment mainly composed of 3-hydroxy acid can be obtained even when the precursor 3HB to be added to the medium is replaced with 3HP. That is, the present invention also makes it possible to obtain P(2HB)-b-P(3HP-ran-4H2MB), P(2HB)-b-P(3HP-ran-24DHB), P(2HB)-b-P(3HP-ran-5HV), P(2HB)-b-P(3HP-ran-6HHx), and the like.

In addition, in the present invention, the synthesis of these polymers depends on the characteristics of the polymerizing enzyme, and if a CoA product of corresponding monomers can be synthesized, it does not matter what the monomer-supplying enzyme is.

### (Example 5)

### <Method for preparing a block copolymer (P(2HB)-b-P(3HB-co-3HV)) from glucose and 2HB>

As shown in Fig. 5A, the present inventors wondered that it would be possible to obtain a block copolymer (P(2HB)-b-P(3HB-co-3HV)) comprising a P(2HB) homopolymer segment and a random copolymer of a segment composed of 3HB and 3HV by culturing a in the presence of glucose and 2HB if the microorganism had a biosynthetic pathway described below.

### <Biosynthetic pathway>

- 2HB is converted to 2HB-CoA in the presence of HadA (CoA transferase).
- Pyruvic acid is generated from glucose in glycolytic system. Acetyl CoA is further generated from pyruvic acid, and is converted to acetoacetyl-CoA in the presence of PhaA, and further 3HB-CoA is biosynthesized in the presence of PhaB (Journal of Biotechnology, 152 (2011), 144-146).
- 2HB is converted to 2-oxobutyric acid in the presence of LdhA(lactate dehydrogenase). Propionic acid and 2-hydroxybutyric acid are generated from threonine and 2-oxobutyric acid (Letters in Applied Microbiology, 1997, 25, 371-374). Propionic acid-CoA is converted from propionic acid through propionic acid-IP. Propionic acid-CoA and acetyl-CoA are converted to 3-ketovaleryl-CoA in the presence of PhaA, and further converted to 3HV-CoA in the presence of PhaB (Journal of Biotechnology, 152 (2011), 144-146).

### <Construction of Ralstonia eutropha-modified strain>

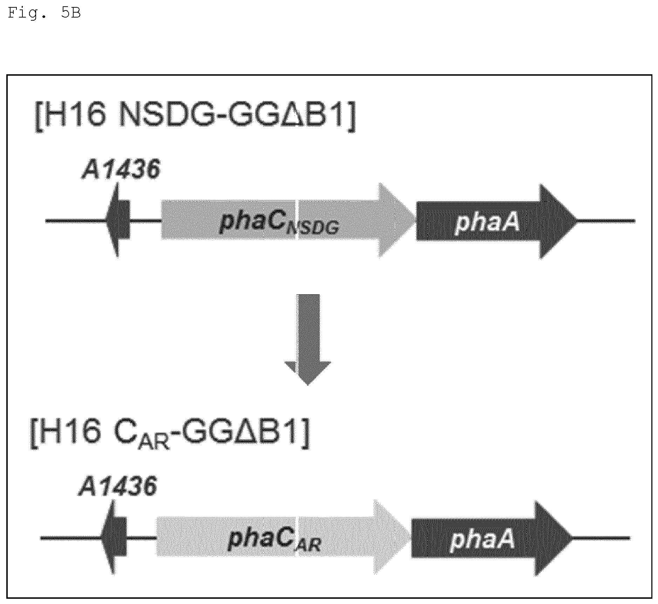

As shown in Fig. 5B, in the pha operon on the chromosome of the R. eutrpha H16 NSDG-GG-ΔB1 strain (see Zhang et al., Microbial Cell Factories, volume 18, Article number: 147(2019)), which is a Ralstonia eutropha glucose-utilizing modified strain, the phaCAR gene was substituted for PhaCNSDG (Aeromonas caviae-derived modified PHA synthase gene) through homologous recombination to construct a PhaC_{AR} expression strain H16 CAR-GG-ΔB1. Specifically, a plasmid pK18mobsacB derivative containing the target fragment was introduced into R. eutrpha through E. coli S17-1 strain having a conjugational transfer ability, a strain in which the phaCAR gene was inserted at a target position on the chromosome DNA was selected based on the kanamycin resistance, and a recombinant strain in which a vector portion was removed was isolated by utilizing fatality in the presence of sucrose of sacB of contained in Subsequently pK18mobsacB.

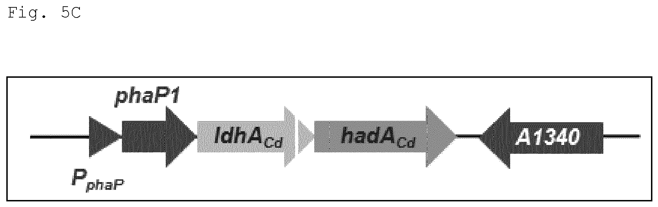

Moreover, as shown in Fig. 5C, Clostridium difficile-derived ldhA (lactate dehydrogenase gene) and hadA (CoA transferase gene) were inserted downstream of phaP1 (PHA granule-binding protein gene) on the chromosome of this modified strain to construct H16 CAR-GG-ΔB1-ldhA hadA which enabled expression of these enzymes under the control of the phaP promoter (increases the transcriptional activity during the synthesis of PphaP and PHA).

### <Culturing of R. eutropha-modified Strain and Analysis on Biosynthesized PHA>

The above modified strain (R. eutrpha H16 CAR-GG-ΔB1-ldhA hadA) was attempted to be cultured by adding 2HB-Na racemate (2HB) to a nitrogen-limited, inorganic salt medium having glucose as a single glucose. As a result, an strong growth inhibition was observed by the addition of 2HB. Study was made on the avoidance of this, it was found that the addition of valine or pyruvic acid was effective. In view of this, in the case where 2HB was added to the medium, valine or pyruvic acid, or both of these was added to conduct culturing. Note that the composition of the nitrogen-limited, inorganic salt medium was 0.9 g of Na₂HPO₄·12H₂O, 0.15 g of KH₂PO₄, 0.05 g of NH₄Cl, 0.02 g of MgSO₄·7H₂O, and 0.1 ml of trace-element solution in 100 ml of water. In addition, the final concentrations of the other components added to the medium were set to 2% (w/v) glucose, 0.25% (w/v) 2HB-Na, 0.8% (w/v) pyruvic acid, and 0.05% valine.

The culturing was conducted in 100 mL scale using a shake flask, and shake-culturing was conducted at 30°C and 118 strokes/min. The bacterial cells were collected from the culture solution after 120 hours by centrifugation and were freeze-dried to obtain dried bacterial cells.

The amount and composition of PHA accumulated in the bacterial cells were analyzed by using gas chromatography (GC) after the PHA contained in the dried bacterial cells was subjected to a methanolysis process. This method is the most general approach used in the analysis of PHA (see PNAS November 11,2008 vol. 105 no. 45 17323 to 17327), and the polymer is completely decomposed into volatile monomer derivatives by heating a PHA solution in the presence of methanol under an acidic condition, making it possible to conduct quantitative and qualitative analyses by using GC. In addition, purified PHA was acquired from the dried bacterial cells by using chloroform extraction and methanol reprecipitation and structural analysis by using ¹H-NMR and molecular weight measurement by using GPC were conducted as in the same methods as described in the above <Analysis on P(3HB-b-(GL-ran-3HB))> and <Gel Filtration Chromatography (GPC) Analysis on P(3HB-b-(GL-ran-3HB))>.

In a nitrogen-limited, inorganic salt medium containing 2% (w/v) glucose, 0.25% (w/v) 2HB-Na, 0.8% (w/v) pyruvic acid, and 0.05% valine, H16 CAR-GG-ΔB1-ldhA hadA strain was cultured, and as a result, 2.24±0.00 g/L dried bacterial cell was obtained, and the PHA amount was 0.42±0.00 g/L (PHA accumulation rate: 18.92±0.20wt%). In the PHA analysis on the dried bacterial cell, it was assumed to be a copolymer composed of 3HB and 2HB (10.60±0.24 mol%).

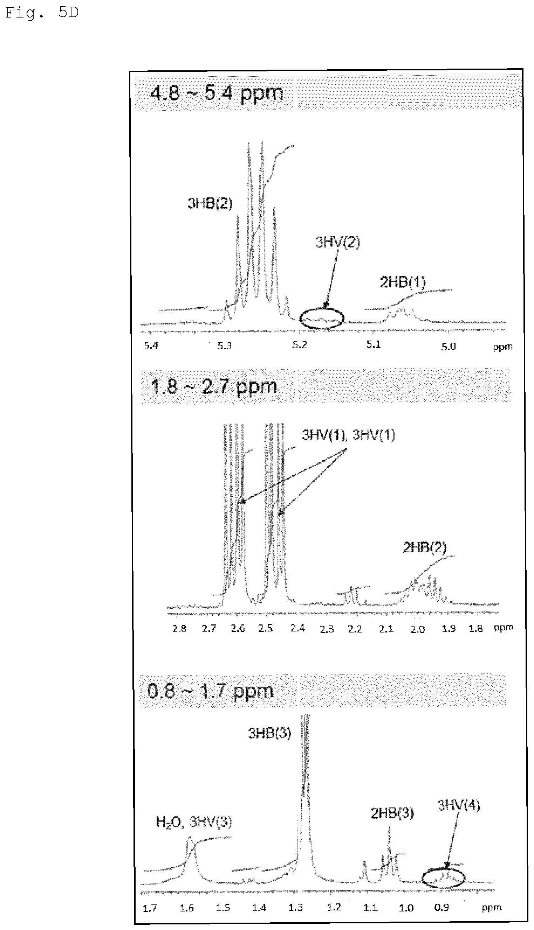

In addition, as a result of the ¹H-NMR analysis, as shown in Fig. 5D, it was revealed that this polymer contained a small amount of 3HV unit in addition to 3HB and 2HB. Moreover, as shown in "4.8 to 5.4 ppm" of Fig. 5D, it was strongly suggested that this polymer was a block copolymer having a homo segment of 2HB.

Moreover, by integrating the results of analysis by using GPC, the detailed composition was determined to be P[(3HB-co-0.5 mol% 3HV)-block-8.3 mol% 2HB]. In addition, the number average molecular weight (Mn)=3.71×10⁵, the weight average molecular weight (Mw)=9.58×10⁵, and the polydispersity index (PDI)=2.58. Furthermore, it was estimated that the average degree of polymerization was 4,311, in which the degree of polymerization of 2HB segment was 358.

### [Industrial Applicability]

As described above, the present invention makes it possible to provide a block copolymer having a homopolymer segment and a copolymer segment. This block copolymer has characteristics of each polymerized segment contained therein and can exhibit various physical properties. Hence, the block copolymer is useful in various industrial fields such as the medical field. In addition, since the present invention makes it possible to provide the block copolymer by using biosynthesis, and the present invention thus can contribute to solution of environmental issues. [Sequence Listing] IBPF21-507WO-J(seq)fin.txt

## Claims

1. A copolymer comprising: a hydroxycarboxylic acid (A) having a hydroxy group only at a 2-position; and a hydroxycarboxylic acid (B) having a hydroxy group at a position other than a 2-position, and having
a homopolymer segment composed of one hydroxycarboxylic acid selected from the group consisting of the hydroxycarboxylic acid (A) and the hydroxycarboxylic acid (B), and
a copolymer segment containing at least two hydroxycarboxylic acids selected from the group consisting of the hydroxycarboxylic acid (A) and the hydroxycarboxylic acid (B),
a segment being a structural unit which constitutes each block in a block copolymer and which is a unit composed of one type of monomer or a unit composed of a plurality of types of monomers.

2. The copolymer according to claim 1, wherein
the hydroxycarboxylic acid (A) is at least one hydroxycarboxylic acid selected from the group consisting of 2-hydroxybutyric acid, 2-hydroxyacetic acid, 2-hydroxypropionic acid, 2-hydroxypentanoic acid, 2-hydroxyhexanoic acid, and 2-hydroxyalkanoic acids derived from amino acids, and
the hydroxycarboxylic acid (B) is at least one hydroxycarboxylic acid selected from the group consisting of 3-hydroxybutyrate, 3-hydroxyhexanoic acid, 3-hydroxypropionic acid, 3-hydroxypentanoic acid, 4-hydroxy-2-methylbutyric acid, 4-hydroxybutyric acid, 2,4-dihydroxybutyric acid, 5-hydroxypentanoic acid, and 6-hydroxyhexanoic acid.

3. The copolymer according to claim 1, wherein
the hydroxycarboxylic acid (A) is 2-hydroxybutyric acid, and the hydroxycarboxylic acid (B) is 3-hydroxybutyrate and 3-hydroxyhexanoic acid, and
the copolymer has a homopolymer segment composed of 2-hydroxybutyric acid and a copolymer segment containing 3-hydroxybutyrate and 3-hydroxyhexanoic acid.

4. The copolymer according to claim 1, wherein
the hydroxycarboxylic acid (A) is 2-hydroxyacetic acid, and the hydroxycarboxylic acid (B) is 3-hydroxybutyrate, and
the copolymer has a homopolymer segment composed of 3-hydroxybutyrate and a copolymer segment containing 2-hydroxyacetic acid and 3-hydroxybutyrate.

5. The copolymer according to claim 1, wherein
the hydroxycarboxylic acid (A) is 2-hydroxyacetic acid, and the hydroxycarboxylic acid (B) is 3-hydroxybutyrate and 3-hydroxyhexanoic acid, and
the copolymer has a homopolymer segment composed of 3-hydroxybutyrate and a copolymer segment containing 2-hydroxyacetic acid, 3-hydroxybutyrate, and 3-hydroxyhexanoic acid.

6. A method for producing the copolymer according to any one of claims 1 to 5, comprising the steps of:
culturing a microorganism expressing a CoA transferase and a polymerizing enzyme in a medium containing a hydroxycarboxylic acid (A) and/or a metabolic precursor thereof and a hydroxycarboxylic acid (B) and/or a metabolic precursor thereof; and
collecting the copolymer from a culture obtained in the culturing step.

## Patentansprüche

1. Copolymer, umfassend: eine Hydroxycarbonsäure (A) mit einer Hydroxygruppe nur an der 2-Stellung; und eine Hydroxycarbonsäure (B) mit einer Hydroxygruppe an einer anderen Position als der 2-Stellung, und Folgendes aufweisend:
ein Homopolymersegment, das aus einer Hydroxycarbonsäure besteht, die ausgewählt ist aus der Gruppe bestehend aus der Hydroxycarbonsäure (A) und der Hydroxycarbonsäure (B), und
ein Copolymersegment, das mindestens zwei Hydroxycarbonsäuren enthält, die ausgewählt sind aus der Gruppe bestehend aus der Hydroxycarbonsäure (A) und der Hydroxycarbonsäure (B),
wobei ein Segment eine Struktureinheit ist, die jeden Block in einem Blockcopolymer bildet und eine Einheit ist, die aus einem Monomertyp oder einer Einheit besteht, die aus einer Vielzahl von Monomertypen besteht.

2. Copolymer nach Anspruch 1, wobei
die Hydroxycarbonsäure (A) mindestens eine Hydroxycarbonsäure ist, die ausgewählt ist aus der Gruppe bestehend aus 2-Hydroxybuttersäure, 2-Hydroxyessigsäure, 2-Hydroxypropionsäure, 2-Hydroxypentansäure, 2-Hydroxyhexansäure und 2-Hydroxyalkansäuren, die von Aminosäuren stammen, und
die Hydroxycarbonsäure (B) mindestens eine Hydroxycarbonsäure ist, die ausgewählt ist aus der Gruppe bestehend aus 3-Hydroxybutyrat, 3-Hydroxyhexansäure, 3-Hydroxypropionsäure, 3-Hydroxypentansäure, 4-Hydroxy-2-methylbuttersäure, 4-Hydroxybuttersäure, 2,4-Dihydroxybuttersäure, 5-Hydroxypentansäure und 6-Hydroxyhexansäure.

3. Copolymer nach Anspruch 1, wobei
die Hydroxycarbonsäure (A) 2-Hydroxybuttersäure ist und die Hydroxycarbonsäure (B) 3-Hydroxybuttersäure und 3-Hydroxyhexansäure ist, und
das Copolymer ein Homopolymersegment, das aus 2-Hydroxybuttersäure besteht, und ein Copolymersegment, das 3-Hydroxybutyrat und 3-Hydroxyhexansäure enthält, aufweist.

4. Copolymer nach Anspruch 1, wobei
die Hydroxycarbonsäure (A) 2-Hydroxyessigsäure ist und die Hydroxycarbonsäure (B) 3-Hydroxybutyrat ist, und
das Copolymer ein Homopolymersegment, das aus 3-Hydroxybutyrat besteht, und ein Copolymersegment, das 2-Hydroxyessigsäure und 3-Hydroxybutyrat enthält, aufweist.

5. Copolymer nach Anspruch 1, wobei
die Hydroxycarbonsäure (A) 2-Hydroxyessigsäure und die Hydroxycarbonsäure (B) 3-Hydroxybutyrat und 3-Hydroxyhexansäure ist, und
das Copolymer ein Homopolymersegment, das aus 3-Hydroxybutyrat besteht, und ein Copolymersegment, das 2-Hydroxyessigsäure, 3-Hydroxybutyrat und 3-Hydroxyhexansäure enthält, aufweist.

6. Verfahren zum Produzieren des Copolymers nach einem der Ansprüche 1 bis 5, umfassend die folgenden Schritte:
Kultivieren eines Mikroorganismus, der eine CoA-Transferase und ein Polymerisierungsenzym exprimiert, in einem Medium, das Folgendes enthält: eine Hydroxycarbonsäure (A) und/oder einen metabolischen Vorläufer davon und eine Hydroxycarbonsäure (B) und/oder einen metabolischen Vorläufer davon; und
Gewinnen des Copolymers aus einer in dem Kultivierungsschritt erhaltenen Kultur.

## Revendications

1. Copolymère comprenant : un acide hydroxycarboxylique (A) présentant un groupe hydroxy uniquement en position 2 ; et un acide hydroxycarboxylique (B) présentant un groupe hydroxy en une position autre qu'une position 2, et présentant
un segment homopolymère composé d'un acide hydroxycarboxylique choisi dans le groupe constitué de l'acide hydroxycarboxylique (A) et de l'acide hydroxycarboxylique (B), et
un segment copolymère contenant au moins deux acides hydroxycarboxyliques choisis dans le groupe constitué de l'acide hydroxycarboxylique (A) et de l'acide hydroxycarboxylique (B),
un segment étant une unité structurelle qui constitue chaque séquence dans un copolymère séquencé et qui est une unité composée d'un seul type de monomère ou une unité composée d'une pluralité de types de monomères.

2. Copolymère selon la revendication 1, dans lequel
l'acide hydroxycarboxylique (A) est au moins un acide hydroxycarboxylique choisi dans le groupe constitué de l'acide 2-hydroxybutyrique, de l'acide 2-hydroxyacétique, de l'acide 2-hydroxypropionique, de l'acide 2-hydroxypentanoïque, de l'acide 2-hydroxyhexanoïque, et d'acides 2-hydroxyalcanoïques dérivés d'acides aminés, et
l'acide hydroxycarboxylique (B) est au moins un acide hydroxycarboxylique choisi dans le groupe constitué du 3-hydroxybutyrate, de l'acide 3-hydroxyhexanoïque, de l'acide 3-hydroxypropionique, de l'acide 3-hydroxypentanoïque, de l'acide 4-hydroxy-2-méthylbutyrique, de l'acide 4-hydroxybutyrique, de l'acide 2,4-dihydroxybutyrique, de l'acide 5-hydroxypentanoïque, et de l'acide 6-hydroxyhexanoïque.

3. Copolymère selon la revendication 1, dans lequel
l'acide hydroxycarboxylique (A) est l'acide 2-hydroxybutyrique, et l'acide hydroxycarboxylique (B) est le 3-hydroxybutyrate et l'acide 3-hydroxyhexanoïque, et
le copolymère présente un segment homopolymère composé d'acide 2-hydroxybutyrique et un segment copolymère contenant du 3-hydroxybutyrate et de l'acide 3-hydroxyhexanoïque.

4. Copolymère selon la revendication 1, dans lequel
l'acide hydroxycarboxylique (A) est l'acide 2-hydroxyacétique, et l'acide hydroxycarboxylique (B) est le 3-hydroxybutyrate, et
le copolymère présente un segment homopolymère composé de 3-hydroxybutyrate et un segment copolymère contenant de l'acide 2-hydroxyacétique et du 3-hydroxybutyrate.

5. Copolymère selon la revendication 1, dans lequel
l'acide hydroxycarboxylique (A) est l'acide 2-hydroxyacétique, et l'acide hydroxycarboxylique (B) est le 3-hydroxybutyrate et l'acide 3-hydroxyhexanoïque, et
le copolymère présente un segment homopolymère composé de 3-hydroxybutyrate et un segment copolymère contenant de l'acide 2-hydroxyacétique, du 3-hydroxybutyrate, et de l'acide 3-hydroxyhexanoïque.

6. Procédé de production du copolymère selon l'une quelconque des revendications 1 à 5, comprenant les étapes suivantes :
la culture d'un micro-organisme exprimant une CoA transférase et une enzyme de polymérisation dans un milieu contenant un acide hydroxycarboxylique (A) et/ou un précurseur métabolique de celui-ci et un acide hydroxycarboxylique (B) et/ou un précurseur métabolique de celui-ci ; et
le recueil du copolymère à partir d'une culture obtenue lors de l'étape de culture.
